# EUROPEAN PATENT SPECIFICATION

(11) **EP 1 210 464 B1**
(45) Date of publication and mention of the grant of the patent: **24.11.2004**
(21) Application number: 00967639.6
(22) Date of filing: 01.09.2000
(51) Int. Cl.: G01N 33/68

(54) **METHODS OF DIAGNOSING OR PROGNOSTICATING AGE-RELATED MACULAR DEGENERATION**
VERFAHREN ZUR DIAGNOSE ODER PROGNOSE VON ALTERSBEDINGTER MACULADEGENERATION
PROCEDE DE DIAGNOSTIC OU DE PRONOSTIC DE LA DEGENERESCENCE MACULAIRE LIEE A L'AGE

(30) Priority: 01.09.1999 EP 99117198; 01.02.2000 EP 00101921
(43) Date of publication of application: 05.06.2002
(73) Proprietor: EVOTEC Neurosciences GmbH, 22525 Hamburg (DE)
(72) Inventor: Richard, Giesbert Universitäts Augenklinik, 20246 Hamburg (DE); Nitsch, Roger, CH-8702 Zollikon (CH)
(74) Representative: Meyers, Hans-Wilhelm, Dr.Dipl.-Chem.
(86) International application number: PCT/EP2000/008554
(87) International publication number: WO 2001/016364

(56) References cited:
- EP-A- 0 330 725
- EP-A- 0 391 714
- WO-A-98/34634
- US-A- 5 231 000
- US-A- 5 270 165
- ABRAHAMSON M ET AL: "MOLECULAR CLONING AND SEQUENCE ANALYSIS OF CDNA CODING FOR THE PRECURSOR OF THE HUMAN CYSTEINE PROTEINASE INHIBITOR CYSTATIN C" FEBS LETTERS,NL,ELSEVIER SCIENCE PUBLISHERS, AMSTERDAM, vol. 216, no. 2, 1 June 1987 (1987-06-01), pages 229-233, XP002038720 ISSN: 0014-5793

## Description

Age-related macular degeneration (AMD) is the most common geriatric eye disorder leading to blindness. Macular degeneration is responsible for visual handicap in what is estimated conservatively to be approximately 16 million individuals worldwide. Among the elderly, the overall prevalence is estimated between 5.7 % and 30 % depending on the definition of early AMD, and its differentiation from features of normal aging, a distinction that remains poorly understood (Ferris et al., Arch. Ophthalmol., 102: 1640 - 1642, 1984; Klein et al., Ophthalmology, 104: 7 - 21, 1997). Histopathologically, the hallmark of early neovascular AMD is accumulation of extracellular drusen and basal laminar deposit (abnormal material located between the plasma membrane and basal lamina of the retinal pigment epithelium) and basal linear deposit (material located between the basal lamina of the retinal pigment epithelium and the inner collageneous zone of Bruch's membrane). The end stage of AMD is characterised by a complete degeneration of the neurosensory retina and of the underlying retinal pigment epithelium in the macular area. Advanced stages of AMD can be subdivided into geographic atrophy and exudative AMD. Geographic atrophy is characterized by progressive atrophy of the retinal pigment epithelium. In exudative AMD the key phenomenon is the occurence of choroidal neovascularisation (CNV). Eyes with CNV have varying degrees of reduced visual acuity, depending on location, size, type and age of the neovascular lesion. The development of choroidal neovascular membranes can be considered a late complication in the natural course of the disease possibly due to tissue disruption (Bruch's membrane) and decompensation of the underlying longstanding processes of AMD.

Many pathophysiological aspects as well as vascular and environmental risk factors are known to be associated with a progression of the disease, but little is known about the etiology of AMD itself as well as about the underlying processes of complications like the occurence of CNV.

Family, twin, segregation, and case-control studies suggest an involvement of genetic factors in the etiology of AMD. However, the extent of heritability, number of genes involved, and mechanisms underlying phenotypic heterogeneity are unknown. The search for genes related to AMD faces challenges: The onset is late in life, and there is usually only one generation available for studies. The parents of patients are often deceased, and the children are too young to manifest the disease. Generally, the heredity of late-onset diseases has been difficult to estimate because of the uncertainties of the diagnosis in previous generations and the inability to diagnose AMD among the children of an affected individual. Even in the absence of the ambiguities in the diagnosis of AMD in previous generations, the late onset of the condition itself, natural death rates, and small family sizes result in underestimation of genetic forms of AMD, and in overestimation of rates of sporadic disease. Moreover, the phenotypic variability is considerable, and it is conceivable that the currently used diagnostic entity of AMD in fact represents a spectrum of underlying conditions with various genetic and environmental factors involved. The search for genetic factors related to AMD has to address these challenges.

Contradictory results were reported for a possible role of *ApoE* polymorphisms in AMD. Some authors have reported a lower frequency of the ε4 allele in subgroups of AMD, while other reports do not confirm this association (Souied et al., Am. J. Ophthalmol., 125: 353 - 359, 1998; De La Paz et al., Invest Ophthalmol. Vis. Sci., 38(3): S796, Abstract nr 3695, 1997). The ε2 allele was reported more frequent in AMD patients (Cruickshanks et al., Invest. Ophthalmol. Vis. Sci., 38(3): S471, Abstract nr 2187, 1997). For instance, also Klaver et al. (Am. J. Human Genet. 63: 200 - 206, 1998) have shown that the apoE gene (APOE) polymorphism is significantly associated with the risk for AMD. The APOE ε4 allele was associated with a decreased risk, and the ε2 allele was associated with a slightly increased risk of AMD. Their results suggest that APOE is a susceptibility gene for AMD.

Further, allelic variations in the *ABCR* gene were proposed to be associated with advanced atrophic AMD (Allikmets et al., Science, 277: 1805 - 1807, 1997), but again, other authors found no evidence to support this hypothesis (Stone et al., Nature Genet., 20: 328 - 329, 1998). Together, these studies illustrate the challenge to identify susceptibility genes in a most likely complex genetic disorder with the influence of unknown extents of environmental factors.

Experimental therapies for AMD have been suggested by Soubrane and Coscas (Wiedemann P., Kohen L. (eds): Macular and retinal diseases. Dev. Ophthalmol. Basel, Karger vol. 29, 77 - 84, 1997). The goal of therapies can be twofold: (i) prevention of the occurence of macular complications or (ii) treatment of the already arised macular complications (central geography atrophy or choroidal neovascularization). One approach for prevention includes antioxidant supplementation with contradictory results regarding the effectiveness. Laser photocoagulation has also been largely ineffective in preventing visual loss in the majority of patients. However, in the therapy of macular complications, laser photocoagulation remains the gold standard for the 15 % of well-defined choroidal new vessels (CNVs). For isolated occult CNVs and vascularized pigment epithelium detachments, no effective treatment is at the horizon. For geographic atrophy, the only hope is at present retinal pigment epithelium and photoreceptors transplantation.

As AMD is an important medical problem, there is a strong need for methods of diagnosing or prognosticating said disease in subjects as well as for methods of treatment. In addition, there is a strong need for identifying inherited risk factors that increase the susceptibility of getting AMD (susceptibility gene).

It was therefore an object of the present invention to provide methods of diagnosing or prognosticating age-related macular degeneration. Another object of the present invention was to provide methods of monitoring the progression of this disease and of evaluating a treatment for it. Still a further object of the present invention was to provide the use of kits in the aforementioned methods.

These objects have been solved by the methods and uses according to the features of the independent claims. The sub-claims define preferred embodiments thereto.

The term "and/or" used in the present specification and in the claims implies that the phrases before and after this term are considered either as alternatives or as a combination. For instance, the wording "determination of a level and/or an activity" means that either only a level, or only an activity, or both a level and an activity are determined.

The term "gene" used in the present specification and in the claims comprises both coding regions (exons) as well as non-coding regions (e.g. non-coding regulatory elements such as a promotor or enhancers; introns; leader & trailer sequences).

In one aspect, the invention features a method for diagnosing or prognosticating age-related macular degeneration in a subject, or determining whether a subject is at increased risk of becoming diseased with age-related macular degeneration. The method includes: determining a level, or an activity, or both said level and said activity, of at least one substance which is selected from the group consisting of a transcription product of a Cystatin C gene, a translation product of a Cystatin C gene, a fragment of said translation product, an amyloid protein, and a transcription product of a gene coding for said amyloid protein in a sample from said subject; and comparing said level, or said activity, or both said level and said activity, of at least one of said substances to a reference value representing a known disease or health status, thereby diagnosing or prognosticating said age-related macular degeneration in said subject, or determining whether said subject is at increased risk of becoming diseased with age-related macular degeneration.

In a further aspect, the invention features a method of monitoring the progression of age-related macular degeneration in a subject. A level, an activity, or both said level and said activity, of at least one substance which is selected from the group consisting of a transcription product of a Cystatin C gene, a translation product of a Cystatin C gene, a fragment of said translation product, an amyloid protein, and a transcription product of a gene coding for an amyloid protein in a sample from said subject is determined. The level and/or activity of at least one of the aforementioned transcription and/or translation products or fragments thereof is compared to a reference value representing a known disease or health status, thereby monitoring the progression of age-related macular degeneration.

In still a further aspect, the invention features a method of evaluating a treatment for age-related macular degeneration, comprising: determining a level, or an activity, or both said level and said activity, of at least one substance which is selected from the group consisting of a transcription product of a Cystatin C gene, a translation product of a Cystatin C gene, a fragment of said translation product, an amyloid protein, and a transcription product of a gene coding for an amyloid protein in a sample obtained from a subject being treated for said age-related macular degeneration. The level and/or activity of at least one of the aforementioned transcription and/or translation products or fragments thereof is compared to a reference value representing a known disease or health status, thereby evaluating the treatment of AMD.

By determining the level and/or activity of the Cystatin C mRNA, a translation product of a Cystatin gene, or a fragment of said translation product (e.g. Cystatin C itself) the activity of the Cystatin C gene can be assessed.

The human Cystatin C gene (*CST3*) maps to chromosome 20p11.2; it contains three exons. Four point mutations in the promoter region of the human Cystatin C gene have been detected by direct sequencing of polymerase chain reaction amplified D N A. The four base changes are all localized within a short segment of 85 base pairs. Three Cystatin C gene alleles could be defined with respect to these promoter mutations. Mendelian inheritance of the polymorphisms has been demonstrated in a study of Caucasian individuals showing frequencies of Cystatin C genotypes AA, BB, CC, AB, AC, and BC. The Sst II polymorphic site within the 5' flanking sequence is in linkage disequilibrium with a second *Sst* II polymorphism within exon 1 of the gene. An Ala/Thr variation in the coding region of the human Cystatin C gene has been detected as said *SSt* II polymorphism. Because of said linkage disequilibrium, the aforementioned polymorphisms in the *CST 3* gene result in the human haplotypes termed *CST 3 A* (nucleotides G, A, and G at positions - 157, -72 and +73), and *CST 3 B* (nucleotides C, C, and A at these positions) (Balbin and Abrahamson, Hum. Genet. 81, 751 - 752, 1991; Balbin et al., Hum. Genet. 92, 206 - 207, 1993; Abrahamson et al., FEBS Lett. 216, 229 - 233, 1987; the contents of these publications are incorporated herein by reference). The open reading frame of *CST3* encodes a 120-residue protein with a molecular mass of 13.3 kDa and a pI of 8.75 (Abrahamson et al., J. Biol. Chem. 261, 11282 - 11289, 1986; Abrahamson et al., FEBS Lett. 216, 229 - 233, 1987; the contents of these publications are incorporated herein by reference). The mature molecule contains intramolecular disulfide bonds, it is partially hydroxylated, no other common post-translational modifications were observed (Grubb and Löfberg, Proc. Natl. Acad. Sci., USA, 79, 3024 - 3027, 1982; Asgeirsson et al., Biochem. J., 329, 497 - 503, 1998; the contents of these publications are incorporated herein by reference). The production rate of Cystatin C is remarkably constant and its plasma concentration can therefore be used as a reliable measure of the glomerular filtration rate (Grubb, Clinical Nephrology, Vol. 38, Suppl. No.1, 20 - 27, 1992). Cystatin C is distributed extensively in the body fluids and is suspected of playing a role in extracellular functions, such as the modulation of inflammatory reactions. It is known to exist in cell types, such as astrocytes, macrophages, and choroid plexus cells. Cystatin C also is a quantitatively dominating cysteine protease inhibitor of cerebrospinal fluid (CSF) whose concentration is five times higher than that of plasma. It binds to and regulates proteolytic activities of cathepsins which have been associated with brain amyloid plaques in Alzheimer's disease, and implicated in the proteolytic processing of the amyloid precursor protein. It has been described that human Cystatin C undergoes dimerization before unfolding. Dimerization leads to a complete loss of its activity as a cysteine proteinase inhibitor (Ekiel et al., J. Mol. Biol. 271, 266 - 277, 1997; the contents of which are incorporated herein by reference). In addition to the termination of biological activity, dimerization and aggregation of Cystatin C is associated with brain amyloid formation in the Islandic form of hereditary cerebral hemorrhage with amyloidosis caused by an inherited mutation (L68Q) within the coding region of *CST3*.

Expression of the human Cystatin C gene leads to a primary transcript containing two intervening sequences. Splicing of this RNA results in a single mRNA species. Translation of the mRNA generates a primary translation product containing a leader sequence in front of the mature Cystatin C amino acid sequence. During secretion of the polypeptide across the membrane of the endoplasmatic reticulum, the leader sequence is cleaved off at the signal peptide cleavage site, giving rise to the secretory Cystatin C molecule. Variations of amino acid positions within the primary translation product, particularly changes at or close to the signal peptide cleavage site, might influence the processing of the precursor protein. Unprocessed or incorrectly processed Cystatin C proteins might cause major irritations of cellular functions, e.g. processing and secretion, and disturb the balance of proteases and protease inhibitors in cells and tissues. This might lead to an increase in the production of amyloidogenic peptides, e.g. by cleavage of amyloid precursor protein (APP) into amyloid β protein. Alternatively, the altered processing of the Cystatin C precursor might result in an increased tendency of the respective protein to aggregate and thereby enhance the aggregation of other amyloidogenic proteins and peptides, e.g. amyloid β protein.

The role of amyloid proteins, in particular amyloid β protein, in neurodegenerative disorders has been extensively described in literature (see e.g. Harper J.D. and Lansbury P.T., Annu. Rev. Biochem., 66, 385 - 407, 1997). The family of Aβ variants is derived from the amyloid precursor protein (APP), a ubiquitous ca. 700-amino acid cell-surface protein. Two variants, Aβ1-40 and Aβ1-42, which differ by truncation at the carboxyl terminus are the predominant amyloid plaque proteins.

Preferred embodiments of the above mentioned methods for diagnosing or prognosticating AMD, or determining an increased risk of becoming diseased with AMD, or monitoring the progression of AMD, or evaluating a treatment of AMD are now disclosed in detail.

Preferably, the Cystatin C gene is a polymorphic variant of the wild-type gene. The presence of at least one B allele, in particular the B/B genotype indicates said subject is at increased risk of developing AMD or indicates diagnosis or prognosis of AMD.

It might be preferred that said subject has previously been determined to have one or more factors indicating that such subject is afflicted with AMD.

In a further preferred embodiment, Aβ1-40 and/or Aβ1-42 are determined as amyloid protein.

In preferred embodiments, the sample is taken from a body fluid, a tissue, or an organ - in particular the eye - of said subject. It is particularly preferred to take a sample from material located between the plasma membrane and basal lamina of the retinal pigment epithelium and/or from material located between the basal lamina of the retinal pigment epithelium and the inner collagenous zone of Bruch's membrane.

According to the present invention, a variation of the level of a translation product of a Cystatin C gene, a fragment thereof, a transcription product of a Cystatin C gene, an amyloid protein, and/or a transcription product of a gene coding for said amyloid protein in said sample from the subject relative to a reference value indicates a diagnosis, or prognosis, or increased risk of said age-related macular degeneration in said subject. As shown in Example 1, a significantly higher frequency of the CST3 BB genotype is observed in patients with exudative AMD. A specific feature associated with this genotype might be an abnormal level of the active form of Cystatin C in specific tissues and body fluids. In a further embodiment, a varied activity of Cystatin C or a translation product of a Cystatin C gene in a sample from a subject relative to a corresponding reference value representing a known health status indicates a diagnosis, or prognosis, or increased risk of AMD in said subject. The varied activity can be due to a polymorphism in the coding region of the Cystatin C gene, leading to a phenotype which differs from the wild-type phenotype. Varied levels of Cystatin C may be due to polymorphisms in the regulatory elements, in particular the promoter, of the Cystatin C gene.

In preferred embodiments, measurement of the level of transcription products of the Cystatin C gene and/or a gene coding for said amyloid protein is performed using Northern blots with probes specific for said genes. Quantitative PCR with primer combinations to amplify gene-specific sequences from cDNA obtained by reverse transcription of RNA extracted from said sample of a subject can also be applied. These techniques are known to those of ordinary skill in the art (see e.g. Watson et al., Rekombinierte DNA, 2nd edition, Spektrum Akademischer Verlag GmbH, Heidelberg, 1993; Watson et al., Recombinant DNA, 2nd ed., W. H. Freeman and Company, 1992).

In preferred embodiments, said level/activity of a translation product of a Cystatin C gene, or fragments thereof - such as Cystatin C -, or said amyloid protein is detected using an immunoassay. These assays can e.g. measure the amount of binding between Cystatin C and an anti-Cystatin C antibody by the use of enzymatic, chromodynamic, radioactive, or luminescent labels which are attached to either the anti-Cystatin C antibody or a secondary antibody which binds the anti-Cystatin C antibody. In addition, other high affinity ligands including cathepsin derivatives may be used. Immunoassays which can be used include e.g. ELISAs, Western blots and other techniques known to those of ordinary skill in the art (see Harlow et al., Antibodies, A Laboratory Manual, Cold Spring Harbor Laboratory Press, Cold Spring Harbor, New York).

The antibody or ligand to be used should preferably specifically detect a translation product of a Cystatin C gene, or a fragment thereof (e. g. Cystatin C) or said amyloid protein. It is preferred that it does not substantially interact with any other protein present in said sample. It is particularly preferred to include specific antibodies or ligands which differentiate monomers from dimers or oligomers of Cystatin C. The detection of the monomeric form of Cystatin C may be more specific to age-related macular degeneration than measuring dimers or oligomers. Measures might be significantly improved with monomer-specific ELISAs.

Monoclonal antibodies capable of recognizing said translation products of the Cystatin C gene, or fragments thereof (e. g. Cystatin C), or said amyloid proteins can be prepared using methods known in the art (see e.g. Köhler and Milstein, Nature 256, 495 - 497 1975; Kozbor et al., Immunol, Today 4, 72, 1983; Cole et al., Monoclonal antibodies and cancer therapy, Alan R. Liss, Inc., pp 77 - 96, 1985; Marks et al., J. Biol. Chem., 16007 - 16010, 1992; the contents of which are incorporated herein by reference). Such monoclonal antibodies or fragments thereof can also be produced by alternative methods known to those of skill in the art of recombinant DNA technology (see e.g. Sastry et al, PNAS 86: 5728, 1989; Watson et al., Rekombinierte DNA, 2nd ed., Spektrum Akademischer Verlag GmbH, 1993; Watson et al, Recombinant DNA, 2nd ed., W. H. Freeman and Company, 1992; the contents of which are incorporated herein by reference). Monoclonal antibodies useful in the methods of the invention are preferably directed to an epitope of Cystatin C or said amyloid protein, such that the complex formed between the antibody and Cystatin C, or between the antibody and said amyloid protein, can be recognized in detection assays. The term "antibodies" encompasses all forms of antibodies known in the art, such as polyclonal, monoclonal, chimeric, recombinatorial, single chain antibodies as well as fragments thereof which specifically bind to a translation product of a Cystatin C gene, a fragment thereof (e.g. Cystatin C or its subfragments), or to an amyloid protein. It is particularly preferred to use specific antibodies that selectively detect Cystatin C monomers, dimers or oligomers, respectively. High-affinity ligands can be prepared by using derivatives of cathepsins which are the natural substrates of Cystatin C biological activity.

It is further preferred to determine e.g. the level of Cystatin C and its activity on basis of an enzymatic assay. As described above, Cystatin C is a cysteine protease inhibitor which binds to and regulates proteolytic activities of cathepsins. A suitable enzymatic assay can therefore be built upon the enzymatic activity of cathepsins indicating the absense or presence of different levels of Cystatin C in its active, monomeric form. It is preferred to use amyloid precursor protein (APP) as a substrate. The generation of A-beta peptides as educts can be measured.

The determination of the level or activity of a translation product of a Cystatin C gene, or a fragment thereof (e.g. Cystatin C), or an amyloid protein, can also be performed on basis of a binding assay. Suitable binding partners include cathepsins or fragments thereof, peptides, peptidomimetics, antibodies and other chemical probes which can specifically recognize the aforementioned substances. It is in general particularly preferred to determine Cystatin C in its monomeric form. Suitable binding partners for an amyloid protein assay include e.g. peptides, peptidomimetics, antibodies and other chemical probes.

If luminescent labels are used in any detection assay, it is preferred to use a confocal optical set-up. It is particularly preferred to conduct detection assays utilising fluorescence techniques known to the person skilled in the art, e. g. fluorescence correlation spectroscopy, FRET, fluorescence anisotropy measurements, fluorescent lifetime measurements, or fluorescence intensity distribution analysis.

In preferred embodiments, the reference value can be that of a level, or an activity, or both said level and said activity, of at least one substance which is selected from the group consisting of a translation product of a Cystatin C gene, a fragment thereof (e. g. Cystatin C), a transcription product of a Cystatin C gene, an amyloid protein, and a transcription product of a gene coding for said amyloid protein in.a sample from a subject not suffering from age-related macular degeneration. The healthy subject can be of the same weight, age, and gender as the subject who is being diagnosed or prognosed for AMD, or for whom an increased risk of becoming diseased with AMD is determined. In some cases, it might be preferred to use a reference value from the subject which is diagnosed.

In preferred embodiments, the subject can be a human, an experimental animal, e. g. a rat or a mouse, a domestic animal, or a non-human primate, e.g. a monkey. The experimental animal can be an animal model for a disorder, e.g. a transgenic mouse with an AMD pathology.

In a preferred embodiment, the level, or the activity, or both said level and said activity, of at least one of said substances in a sample is determined at least twice, e.g. at two points which are weeks or months apart. The levels or activities at these two time points are compared in order to monitor the progression of AMD. It might further be preferred to compare a level, or an activity, or both said level and said activity of at least one substance which is selected from the group consisting of a translation product of a Cystatin C gene, a fragment thereof (e. g. Cystatin C or its subfragments), a transcription product of a Cystatin C gene, an amyloid protein and a transcription product of a gene coding for said amyloid protein in said sample with a level, an activity, or both said level and said activity, of at least one of said substances in a series of samples taken from said subject over a period of time. In further preferred embodiments, said subject receives a treatment prior to one or more of said sample gatherings. Preferably, said level, or said activity, or said level and said activity, is determined before and after a treatment for AMD is administered to said subject. This procedure is suitable for evaluating a treatment of AMD.

In another aspect, the invention features, a method of diagnosing or prognosticating age-related macular degeneration in a subject, or determining whether a subject is at increased risk of becoming diseased with age-related macular degeneration. The method includes: determining the presence or absense of a mutation or polymorphism in a Cystatin C gene in a sample from said subject, thereby diagnosing, or prognosticating, or determining an increased risk of AMD in said subject. The term "gene" as used in the present specification comprises coding as well as non-coding regions, e.g. non-coding regulatory elements such as a promotor or enhancer sequences. Such a mutation can e.g. be a substitution, deletion or addition of at least one base. Such mutations may result in "mis-sense" information or in "non-sense" information associated with a termination codon or a frame shift. Polymorphisms and allele variations occur more frequently in the general population but induce in principle the identical genetic alterations. In case of polymorphisms or mutations in the coding region of the gene, the translation product or processed peptides/proteins thereof may have an amino acid sequence which differs from that of the translation products or processed peptides/proteins thereof in their wild-type form. In case of a polymorphic phenotype, usually a control subject with a wild-type Cystatin C gene will be chosen. In this control subject, one will therefore determine only wild-type Cystatin C. However, polymorphisms might also be extant in regions preceding and/or following the coding region (leader and trailer) or in intervening sequences (introns) between individual coding segments (exons). Such mutations or polymorphisms may be found within the promoter region, an example of which is the *Sst* II polymorphic site in the promoter region of the human Cystatin C gene (*CST 3*).

It is preferred to determine the presence of a B allele in the Cystatin C gene. The human Cystatin C gene, called *CST3,* has been sequenced and its A and B alleles were also described (Balbin et al., Biol. Chem. Hoppe-Seyler, Vol. 373, 471 - 476, 1992; Abrahamson et al., Biochem. J. 268, 287 - 294, 1990; Abrahamson et al., Hum. Genet. 82, 223 - 226, 1989; the contents of these publications are incorporated herein by reference). The presence of at least one B allele in said Cystatin C gene indicates said subject and potentially its descendants are at increased risk of developing age-related macular degeneration. In particular, homozygous *CST* 3 B/B subjects are at increased risk of developing AMD. The data shown in Example 1 indicate an association of *CST3* B/B genotype with AMD. In a further preferred embodiment, a disease-predisposing mutation identifiable by linkage analysis of the mutations in said B allele might also indicate that the subject under study is at increased risk of developing AMD.

Determining the presence or absense of a mutation or polymorphism in a Cystatin C gene in a sample from said subject may comprise determining a partial nucleotide sequence of the DNA from said subject, said partial nucleotide sequence indicating the presence or absence of said mutation or polymorphism. It may further be preferred to perform a polymerase chain reaction with the DNA from said subject and subsequent restriction analysis to determine the presence or absence of said mutation or polymorphism. Suitable primers can be used for amplifying the promoter region as well as the coding sequence of exon 1 of the human Cystatin C gene in order to subsequently analyse the *Sst* II polymorphic sites herein. For the determination of mutations or polymorphisms in the Cystatin C gene, it is preferred to use DNA from body cells, in particular white blood cells.

In another preferred embodiment, the method further includes: determining a level, or an activity, or both said level and said activity, of at least one substance which is selected from the group consisting of a translation product of a Cystatin C gene, a fragment of said translation product (e.g. Cystatin C), a transcription product of a Cystatin C gene, an amyloid protein, and a transcription product of a gene coding for said amyloid protein in a sample from said subject; and comparing said level, or said activity, or both said level and said activity, of at least one of said substances to a reference value. A variation (in particular an increase) of a level of a translation product of a Cystatin C gene, a fragment thereof (e. g. Cystatin C or its subfragments), or a transcription product of a Cystatin C gene in a sample from a patient relative to a reference value representing a known health status indicates a diagnosis, or prognosis, or increased risk of AMD in the patient. In a further embodiment, a varied activity of Cystatin C or a varied processing of the Cystatin C precursor proteins (i.e. the translation products of the Cystatin C gene) in a sample from the patient relative to a reference value representing a known health status also indicates a diagnosis, or prognosis, or increased risk of AMD. The varied activity/processing can be due to a polymorphism in the coding region of the Cystatin C gene, leading to a phenotype which differs from the wild-type phenotype.

In another aspect, the invention features, the use of a kit for diagnosis, or prognosis of age-related macular degeneration, or for determination of increased risk of developing AMD, or for monitoring a progression of age-related macular degeneration in a subject, or for monitoring success or failure of a therapeutic treatment of said subject, said kit comprising at least one reagent which is selected from the group consisting of (i) reagents that selectively detect a transcription product a Cystatin C gene, (ii) reagents that selectively detect a translation product of a Cystatin C gene and/or a processed or fragmented peptide of the translation product, (iii) reagents that selectively detect a mutation or polymorphism in a Cystatin C gene, and (iii) reagents that selectively detect a transcription product and/or a translation product of a gene coding for an amyloid protein. Preferably, it further comprises instructions for use. Processing of the translation product might lead to the natural Cystatin C, however also mis-processing might take place, leading to fragments of the translation product which differ from the normally occurnng Cystatin C. The use preferably comprises (i) detecting a level, or an activity, or both said level and said activity, of said Cystatin C, or of said transcription/translation products of said Cystatin C gene, or of said amyloid protein, or of said transcription products of a gene coding for an amyloid protein, in a sample from said subject; and/or (ii) detecting a presence or absence of mutations or polymorphisms in said Cystatin C gene in a sample from said subject. A varied level, or activity, or both said level and said activity, of at least one of the aforementioned substances, compared to a reference value representing a known health status indicates a diagnosis, or prognosis, or an increased risk of developing AMD. Also the presence of a mutation or polymorphism in said Cystatin C gene indicates a diagnosis, or prognosis, or an increased risk of developing age-related macular degeneration.

It is preferred that said at least one reagent and said instructions are packaged in a single container. In preferred embodiments, said reference value is that of a level, or an activity, or both said level and said activity, of at least one substance which is selected from the above mentioned group in a sample from a subject not suffering from said AMD. The healthy subject can be of the same weight, age, and gender as the subject who is being diagnosed, or prognosed for AMD, or for whom an increased risk of developing AMD is determined. In some cases, it might be preferred to use a reference value of the subject which is to be diagnosed. Said kit suitable for commercial manufacture and sale can still further include appropriate standards, positive and negative controls.

The kit preferably comprises reagents that selectively detect the presence of at least one B allele in said Cystatin C gene, in particular the presence of the B/B genotype. This indicates a diagnosis, or prognosis, or an increased risk of age-related macular degeneration. In order to exclude a false positive diagnosis, it should be remarked that a mutation or polymorphism in the Cystatin C gene in the codon for leucine at position 68 which abolishes an *A*/*u*I restriction site is not indicative for age-related macular degeneration, but for hereditary Cystatin C amyloid angiopathy.

Determining the presence or absense of a mutation or polymorphism in a Cystatin C gene in a sample from said subject may comprise determining a partial nucleotide sequence of the DNA from said subject, said partial nucleotide sequence indicating the presence or absence of said mutation or polymorphism. It may further be preferred to perform a polymerase chain reaction with the DNA from said subject and subsequent restriction analysis to determine the presence or absence of said mutation. Therefore, in a preferred embodiment, said kit comprises primers for amplifying at least parts of the promoter region and/or of the coding region of a Cystatin C gene in order to subsequently analyze the Sst II (or isoenzyme) polymorphic sites herein.

In preferred embodiments, the constituents of said kit allow for measurement of the level of transcription products of the Cystatin C gene, or of a gene coding for an amyloid protein. This measurement is e.g. performed using Northern blots with probes specific for said genes. Quantitative PCR with primer combinations to amplify gene-specific sequences from cDNA obtained by reverse transcription of RNA extracted from body cells of a subject can also be applied. These techniques are known to those of ordinary skill in the art (see e.g. Watson et al., Rekombinierte DNA, 2nd edition, Spektrum Akademischer Verlag GmbH, Heidelberg, 1993; Watson et al., Recombinant DNA, 2nd ed., W. H. Freeman and Company, 1992).

For the determination of mutations or polymorphisms in the Cystatin C gene, it is preferred to use DNA from body cells including fibroblasts and white blood cells. For the other analyses, it is particularly preferred to use samples taken from the eye, as explained above.

In preferred embodiments, the constituents of said kit allow for the detection of said level and/or activity of said above mentioned substances using an immunoassay. These assays can e.g. measure the amount of binding between Cystatin C and an anti-Cystatin C antibody by the use of enzymatic, chromodynamic, radioactive, or luminescent labels which are attached to either the anti-Cystatin C antibody or a secondary antibody which binds the anti-Cystatin C antibody. In addition, other high affinity ligands including cathepsin derivatives may be used. Immunoassays which can be used include e.g. ELISAs, Western blots and other techniques known to those of ordinary skill in the art (see Harlow et al., Antibodies, A Laboratory Manual, Cold Spring Harbor Laboratory Press, Cold Spring Harbor, New York).

The antibody or ligand to be used should preferably specifically detect a translation product of a Cystatin C gene, a fragment of said translation product (e. g. Cystatin C), or an amyloid protein. It is preferred that the antibody or ligand does not substantially interact with any other protein present in said sample. It is particularly preferred to include in said kit specific antibodies or ligands which differentiate monomers from dimers or oligomers of Cystatin C.

The detection of the monomeric form of Cystatin C may be more specific to AMD than measuring dimers or oligomers. Measures might be significantly improved with monomer-specific ELISAs.

Monoclonal antibodies capable of recognizing a translation product of a Cystatin C gene, a processed peptide thereof (e. g. Cystatin C), or an amyloid protein can be prepared using methods known in the art (see e.g. Köhler and Milstein, Nature 256, 495 - 497 1975; Kozbor et al., Immunol. Today 4, 72, 1983; Cole et al., Monoclonal antibodies and cancer therapy, Alan R. Liss, Inc., pp 77 - 96, 1985; Marks et al., J. Biol. Chem., 16007 - 16010, 1992; the contents of which are incorporated herein by reference). Such monoclonal antibodies or fragments thereof can also be produced by alternative methods known to those of skill in the art of recombinant DNA technology (see e.g. Sastry et al, PNAS 86: 5728, 1989; Watson et al., Rekombinierte DNA, 2nd ed., Spektrum Akademischer Verlag GmbH, 1993; Watson et al., Recombinant DNA, 2nd ed., W. H. Freeman and Company, 1992; the contents of which are incorporated herein by reference). Monoclonal antibodies useful in the kit of the invention are preferably directed to an epitope of Cystatin C or an amyloid protein, such that the complex formed between the antibody and Cystatin C, or between the antibody and said amyloid protein, can be recognized in detection assays. The term "antibodies" encompasses all forms of antibodies known in the art, such as polyclonal, monoclonal, chimeric, recombinatorial, single chain antibodies as well as fragments thereof which specifically bind to a translation product of a Cystatin C gene, a processed peptide thereof such as Cystatin C, or to an amyloid protein. It is particularly preferred to use specific antibodies that selectively detect Cystatin C monomers, dimers or oligomers, respectively. High-affinity ligands can be prepared by using derivatives of cathepsins which are the natural substrates of Cystatin C biological activity.

It is further preferred that the kit comprises constituents which allow to determine the level of Cystatin C and its activity on basis of an enzymatic assay. As described above, Cystatin C is a cysteine protease inhibitor which binds to and regulates proteolytic activities of cathepsins. A suitable enzymatic assay can therefore be built upon the enzymatic activity of cathepsins indicating the absense or presence of different levels of Cystatin C in its active, monomeric form. It is preferred to use amyloid precursor protein (APP) as a substrate. The generation of A-beta peptides as educts can be measured.

The determination of the level or activity of a translation product of a Cystatin C gene, a fragment thereof (e. g. Cystatin C), or an amyloid protein, can also be performed on basis of a binding assay. Suitable binding partners include cathepsins or fragments thereof, peptides, peptidomimetics, antibodies and other chemical probes which can specifically recognize said translation product or Cystatin C. It is in general particularly preferred to determine Cystatin C in its monomeric form. Suitable binding partners for an amyloid protein assay include e.g. peptides, peptidomimetics, antibodies and other chemical probes. The aforementioned binding partners are preferably constituents of a kit according to the present invention.

Although not within the scope of the claims, the present invention enables a method of treating or preventing AMD in a subject comprising administering to said subject in a therapeutically effective amount an agent or agents which directly or indirectly modulates a biological activity, or level, or both said activity and level, of at least one substance which is selected from the group consisting of a Cystatin C gene, a transcription product of a Cystatin C gene, a translation product of a Cystatin C gene, a processed/fragmented peptide thereof (e. g. Cystatin C), a gene coding for an amyloid protein, a transcription product of a gene coding for an amyloid protein, and an amyloid protein.

It is preferred that said agent or agents reduce a biological activity, or level, or both said activity and level, of at least one of the mentioned substances.

In preferred embodiments, said agents bind or inhibit Cystatin C, as e.g. cathepsin derivatives or Cystatin C analoga. In further preferred embodiments, said agents inhibit the formation of macular plaques, in particular drusen or amyloid plaques, in said subject's eyes.

In preferred embodiments, the method comprises the application of per se known methods of gene therapy and/or antisense nucleic acid technology to administer said agent or agents.

In general, gene therapy includes several approaches: molecular replacement of a mutated gene, addition of a new gene resulting in the synthesis of a therapeutic protein, and modulation of endogeneous cellular gene expression by drugs. Gene-transfer techniques are described in detail (see e.g. Behr, Acc. Chem. Res. 26, 274 - 278, 1993; Mulligan, Science 260, 926 - 931, 1993; the contents of which are incorporated herein by reference) and include direct gene-transfer techniques such as mechanical microinjection of DNA into a cell as well as indirect techniques employing biological vectors (like recombinant viruses, especially retroviruses) or model liposomes, or techniques based on transfection with DNA coprecipitation with polycations, cell membrane perturbation by chemical (solvents, detergents, polymers, enzymes) or physical means (mechanic, osmotic, thermic, electric shocks).

In particular, and although not within its scope, the invention enables a method of treating or preventing age-related macular degeneration by means of antisense nucleic acid therapy, i.e. the down-regulation of an inappropriately expressed or defective gene by the introduction of antisense nucleic acids or derivatives thereof into certain critical cells (see e.g. Gillespie, DN & P 5(7), 389 - 395, 1992; Agrawal, Tibtech 13, 197 - 199, 1995; Crooke, Bio/Technology 10, 882 - 886, 1992; the contents of which are incorporated herein by reference). Apart from hybridization strategies, the application of ribozymes, i.e. RNA molecules that act as enzymes, destroying RNA that carries the message of disease has also been described (see e.g. Barinaga, Science, 262, 1512 - 1514, 1993; the contents of which are incorporated herein by reference). In preferred embodiments, the subject to be treated is a human, and therapeutic antisense nucleic acids or derivatives thereof are directed against the human Cystatin C gene *CST-3*, or transcription products of *CST-3*. Cell penetration can be performed by known strategies such as coupling of antisense nucleic acids and derivatives thereof to carrier particles, or the above described techniques. Strategies for administering targeted therapeutic oligodeoxynucleotides are known to those of skill in the art (see e.g. Wickstrom, Tibtech, 10, 281 - 287, 1992; the contents of which are incorporated herein by reference). In some cases, delivery can be performed by mere topical application. Further approaches are directed to intracellular expression of antisense RNA. In this strategy, cells are transformed *ex vivo* with a recombinant gene that directs the synthesis of an RNA that is complementary to a region of the target nucleic acid. Therapeutically use of intracellularly expressed antisense RNA is procedurally similar to gene therapy.

In preferred embodiments, the method comprises grafting donor cells into the eye of said subject, said subject or donor cells preferably treated so as to minimise or reduce graft rejection, wherein said donor cells are genetically modified by insertion of at least one transgene encoding said agent or agents. Said transgene might be carried by a viral vector, in particular a retroviral vector. The transgene can be inserted into the donor cells by a nonviral physical transfection of DNA encoding a transgene, in particular by microinjection. Insertion of the transgene can also be performed by electroporation, chemically mediated transfection, in particular calcium phospate transfection, liposomal mediated transfection, etc.

In preferred embodiments, said agent is a therapeutic protein which can be administered to said subject, preferably a human, by a process comprising introducing subject cells into said subject, said subject cells having been treated *in vitro* to insert a DNA segment encoding said therapeutic protein, said subject cells expressing *in vivo* in said subject a therapeutically effective amount of said therapeutic protein. Said DNA segment can be inserted into said cells *in vitro* by a viral vector, in particular a retroviral vector.

In preferred embodiments, the therapeutic nucleic acid or protein reduces amyloid formation by interacting with a Cystatin C gene, its transcription products, a translation product of a Cystatin C gene, or a processed peptide thereof such as Cystatin C. Sald amyloid is e.g. β-amyloid derived by proteolytic processing of the amyloid precursor protein (APP) known in Alzheimer's disease.

In preferred embodiments, the subject can be a human, an experimental animal, e.g. a rat or a mouse, a domestic animal, or a non-human primate, e.g. a monkey. The experimental anlmal can be an animal model for a disorder, e.g. a transgenic mouse with an AMD pathology.

Not within the scope of the claims is a modulator of an activity, or level, or both said activity and level, of at least one substance which is selected from the group consisting of a Cystatin C gene, a transcription product of a Cystatin C gene, a translation product of a Cystatin C gene, a processed/fragmented peptide thereof (e. g. Cystatin C), a gene coding for an amyloid protein, a transcription product of a gene coding for an amyloid protein, and an amyloid protein. This modulator or agent might directly or indirectly affect a biological activity, or level, or both said activity and level, of at least one of the aforementioned substances.

It is preferred that said modulator(s) reduce(s) a biological activity, or level, or both said activity and level, of at least one substance which is selected from the above mentioned substances. In preferred embodiments, the agent is a therapeutic nucleic add or protein which reduces amyloid formation by interacting with a Cystatin C gene, its transcription/translation products, or Cystatin C. It might also interact with an amyloidic peptide, preferably β-amyloid derivable by proteolytic processing of the amyloid precursor protein (APP) known in Alzheimer's disease. A medicament may comprise a modulator of biological activity, or level, or both said activity and level, of at least one substance which is mentioned above. It is preferred that said modulator reduces a biological activity, or level, or both said activity and level, of at least one of said substances.

An agent/modulator which directly or indirectly affects a biological activity, or level, or both said activity and level, of at least one substance may be selected from the group consisting of a Cystatin C gene, a transcription product of a Cystatin C gene, a translation product of a Cystatin C gene, a processed/fragmented translation product such as e. g. Cystatin C, a gene coding for an amyloid protein, a transcription product of a gene coding for an amyloid protein, and an amyloid protein, for treating or preventing age-related macular degeneration. It is preferred that said agent(s) reduce(s) a biological activity, or level, or both said activity and level, of at least one of said substances. In preferred embodiments, the agent is a therapeutic nucleic acid or protein which reduces amyloid formation by interacting with a Cystatin C gene, its transcription products, or a un/processed translation product of a Cystatin C gene. If the modulator is capable of interacting with an amyloidic protein, this amyloid is preferably β-amyloid derivable by proteolytic processing of the amyloid precursor protein (APP) known in Alzheimer's disease.

A modulator of a biological activity, or level, or both said activity and level, of at least one substance which is selected from the group consisting of a Cystatin C gene, a transcription product of a Cystatin C gene, an (un)processed translation product of a Cystatin C gene, a gene coding for an amyloid protein, a transcription product of a gene coding for an amyloid protein, and an amyloid protein, may be used for a preparation of a medicament for treating or preventing age-related macular degeneration. It is preferred that said modulator(s) reduce(s) a biological activity, or level, or both said activity and level, of at least one of said substances. In preferred embodiments, the agent is a therapeutic nucleic acid or protein which reduces amyloid formation by interacting with a Cystatin C gene, its transcription products, or the processed translation product Cystatin C. It is preferred that it interacts with β-amyloid which is derivable by proteolytic processing of the amyloid precursor protein (APP) known in Alzheimer's disease.

In another aspect, but not within its scope the invention enables a method for identifying an agent that affects age-related macular degeneration, comprising the steps of:
- providing a sample containing at least one substance which is selected from the group consisting of a Cystatin C gene, a transcription product of a Cystatin C gene, an (un)processed translation product of a Cystatin C gene, a gene coding for an amyloid protein, a transcription product of a gene coding for an amyloid protein, and an amyloid protein;
- contacting said sample with at least one agent; and
- comparing an activity, or level, or both said activity and level, of at least one of said substances before and after said contacting.

It is preferred that said agent reduces an activity, or level, or both said activity and level, of at least one of said substances.

In preferred embodiments, measurement of the level of transcription products of a Cystatin C gene, or of a gene coding for an amyloid protein, is performed using Northern blots with probes specific for said genes. Quantitative PCR with primer combinations to amplify gene-specific sequences from cDNA obtained by reverse transcription of RNA extracted from a subject can also be applied. These techniques are known to those of ordinary skill in the art (see e.g. Watson et al., Rekombinierte DNA, 2nd edition, Spektrum Akademischer Verlag GmbH, Heidelberg, 1993; Watson et al., Recombinant DNA, 2nd ed., W. H. Freeman and Company, 1992).

In preferred embodiments, said level or activity of a translation product of a Cystatin C gene, of a processed peptide thereof such as Cystatin C, or of an amyloid protein, is detected using an immunoassay. These assays can e.g. measure the amount of binding between Cystatin C and an anti-Cystatin C antibody by the use of enzymatic, chromodynamic, radioactive, or luminescent labels which are attached to either the anti-Cystatin C antibody or a secondary antibody which binds the anti-Cystatin C antibody. In addition, other high affinity ligands including cathepsin derivatives may be used. Immunoassays which can be used include e.g. ELISAs, Western blots and other techniques known to those of ordinary skill in the art (see Harlow et al., Antibodies, A Laboratory Manual, Cold Spring Harbor Laboratory Press, Cold Spring Harbor, New York).

The antibody or ligand to be used should preferably specifically detect Cystatin C or an amyloid protein. It is preferred that it does not substantially interact with any other protein present in said sample. It is particularly preferred to include specific antibodies or ligands which differentiate monomers from dimers or oligomers of Cystatin C.

Monoclonal antibodies capable of recognising a translation product of a Cystatin C gene, a processed peptide thereof (e. g. Cystatin C), or an amyloid protein, can be prepared using methods known in the art (see e.g. Köhler and Milstein, Nature 256, 495 - 497 1975; Kozbor et al., Immunol. Today 4, 72, 1983; Cole et al., Monoclonal antibodies and cancer therapy, Alan R. Liss, Inc., pp 77 - 96, 1985; Marks et al., J. Biol. Chem., 16007 - 16010, 1992; the contents of which are incorporated herein by reference). Such monoclonal antibodies or fragments thereof can also be produced by alternative methods known to those of skill in the art of recombinant DNA technology (see e.g. Sastry et al, PNAS 86: 5728, 1989; Watson et al., Rekombinierte DNA, 2nd ed., Spektrum Akademischer Verlag GmbH, 1993; Watson et al., Recombinant DNA, 2 nd., W. H. Freeman and Company, 1992; the contents of which are incorporated herein by reference). Monoclonal antibodies useful in the methods of the invention are preferably directed to an epitope of Cystatin C or an amyloid protein, such that the complex formed between the antibody and Cystatin C, or between the antibody and said amyloid protein, can be recognised in detection assays. The term "antibodies" encompasses all forms of antibodies known in the art, such as polyclonal, monoclonal, chimeric, recombinatorial, single chain antibodies as well as fragments thereof which specifically bind to a translation product of a Cystatin C gene, a fragment thereof (e. g. Cystatin C), or said amyloid protein. It is particularly preferred to use specific antibodies that selectively detect Cystatin C monomers, dimers or oligomers, respectively. High-affinity ligands can be prepared by using derivatives of cathepsins which are the natural substrates of Cystatin C biological activity.

It is further preferred to determine the level of an (un)processsed translation product of a Cystatin C gene, e. g. Cystatin C, and its activity on basis of an enzymatic assay. As described above, Cystatin C is a cysteine protease inhibitor which binds to and regulates proteolytic activities of cathepsins. A suitable enzymatic assay can therefore be built upon the enzymatic activity of cathepsins indicating the absense or presence of different levels of Cystatin C in its active, monomeric form. It is preferred to use amyloid precursor protein (APP) as a substrate. The generation of A-beta peptides, in particular A-beta 40 and A-beta 42 peptides, as educts can be measured.

The determination of the level or activity of an unprocessed or processed translation product of a Cystatin C gene, or an amyloid protein, can also be performed on basis of a binding assay. Suitable binding partners include cathepsins or fragments thereof, peptides, peptidomimetics, antibodies and other chemical probes which can specifically recognise Cystatin C, or a precursor or fragment thereof. It is in general particularly preferred to determine Cystatin C in its monomeric form. Suitable binding partners for an amyloid protein assay include e.g. peptides, peptidomimetics, antibodies and other chemical probes.

If luminescent labels are used in any detection assay, it is preferred to use a confocal optical set-up. Preferably fluorescence techniques can be used, e.g. fluorescence correlation spectroscopy, fluorescence intensity distribution analysis, fluorescence lifetime analysis, FRET, or fluorescence anisotropy measurements.

The accompanying figures illustrate the present invention. Other features and advantages of the invention will be apparent from the following detailed description of the examples, and from the claims.

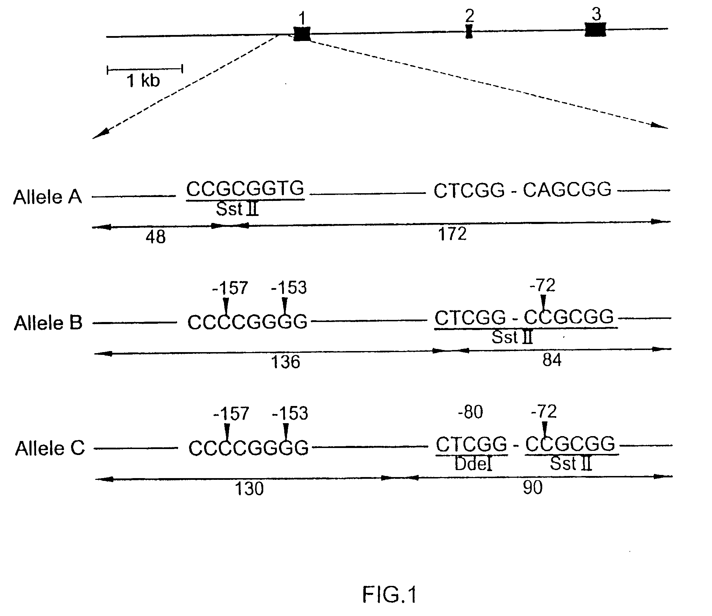

**Figure 1** depicts a schematic illustration of the human Cystatin C gene with the exons numbered and shown as filled boxes as well as the three different alleles (A, B and C) with their respective nucleotide sequences given around the mutations in the promotor region. Nucleotide numbering for the base substitutions relates to the start site for Cystatin C translation (+1 - +3 equals initiator methionine codon). The polymorphic *Sst* II and *Dde* I sites are underlined, and expected lengths of DNA fragments after respective cleavage are indicated. Please note that the *Sst* II polymorphic site within the 5' flanking sequence is in linkage disequilibrium with a second *Sst* II polymorphism within exon 1 of the gene (not shown). An Ala/Thr variation in the coding region of the human Cystatin C gene has been detected as said *SSt* II polymorphism. Because of said linkage disequilibrium, the aforementioned polymorphisms in the *CST 3* gene result in the human haplotypes termed *CST 3 A* (nucleotides G, A, and G at positions -157, -72 and +73), and *CST 3 B* (nucleotides C, C, and A at these positions).

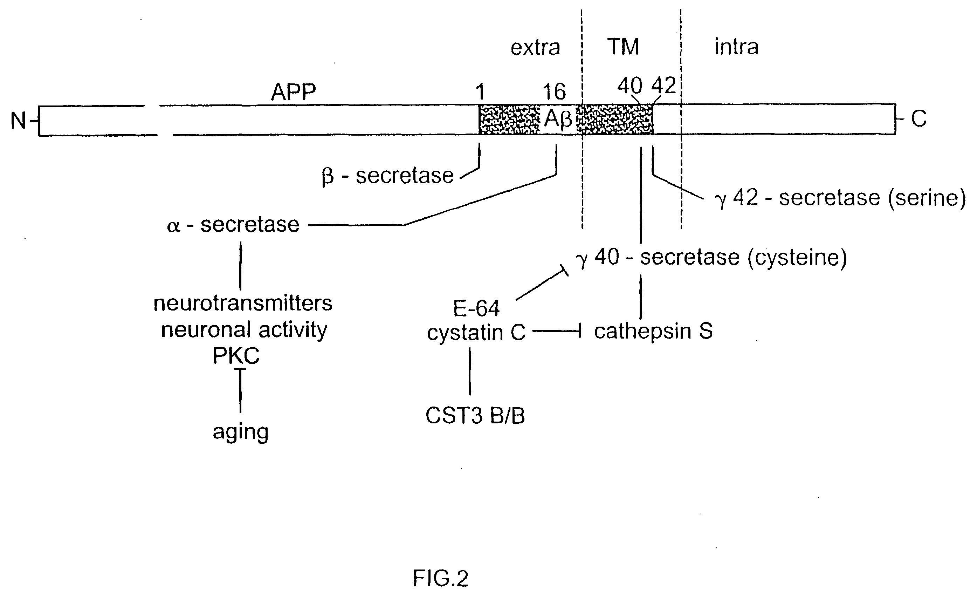

**Figure 2** depicts the possible roles of Cystatin C and the *Aspergillus japonicus* cysteine proteinase inhibitor E-64 in the amyloid precursor protein (APP) processing and generation of the amyloid β peptide (A_{β}).

**Figure 3** depicts a Kaplan-Meier Hazard Function: Cumulative Hazard dependent on age and *CST3* genotype. For further details see EXAMPLE 2.

### EXAMPLE 1

### Patients and Methods

Between February and October 1998, 167 patients with the clinical diagnosis of exudative AMD of all types, classic CNV, occult CNV and pigment epithelial detachment were recruited at the University Eye Hospital Hamburg-Eppendorf, Germany. 268 unrelated age-matched control subjects were randomly selected to represent a sample of the general population, and therefore can be expected to develop AMD at the population rate (Table 1). The possibility of selecting elderly control subjects without signs of AMD was not chosen because this would require the differentiation of features associated with normal aging from early AMD. This distinction is difficult and its theoretical basis is weak. Informed consent was obtained from all patients and controls. This study was conducted according to the tenets of the Declaration of Helsinki and was approved by our institutional human experimentation committee. Inclusion criteria for patients were: Diagnosis of uni- or bilateral neovascular AMD by using fluorescein angiography and absence of other retinal dystrophies or diseases that may be associated with the development of CNV.
A comprehensive ophthalmologic examination which included visual acuity measurement, fundus examination and fluorescein angiography was done in patients. Age at presentation and gender were recorded for both patients and control subjects. No age of onset of AMD was recorded for patients because due to the lingering course of the early forms often no definite date can be given.

Fluorescein angiographic photographs were evaluated according to the guidelines of the international classification and grading system of the international ARM epidemiological study group by two independent graders. Grading results were subsequently reviewed by another independent grader (The international ARM Epidemiological Study Group. An international classification and grading system for age-related maculopathy and age-related macular degeneration. Surv. Ophthalmol. 39(5): 367 - 374, 1995). Inclusion criteria for the patient group were the presence of any form of neovascular maculopathy secondary to age-related macular degeneration including classic CNV, occult CNV, pigment epithelial detachment, or a combination of any of the above in one or both eyes. If only one eye was affected by neovascular AMD, the other eye had to present with phenomena of early AMD including more than 5 hard drusen, soft drusen, pigmentary abnormalities, or with advanced atrophic AMD (geographic atrophy) to ensure that AMD was the cause of neovascular maculopathy. In cases of disagreement between the two initial graders the conclusive grading was done by a the reviewing grader. To avoid bias, all graders were blinded with respect to the results of the genotype analyses.

Blood was collected from peripheral veins into EDTA tubes from patients and control subjects. Genomic DNA was extracted from isolated leukocytes using a standard salt precipitation technique and its concentration was determined spectrophotometrically. The PCR product was generated using the primers 024, TGGGAGGGACGAGGCGTTCC and 1206R, TCCATGGGGCCTCCCACCAG. A 10 µl polymerase chain reaction was performed, containing 0.4 µl of suspended genomic DNA, 500 nM of each forward and reverse primer, 1.5 mM magnesium chloride, 1 µl 10x buffer, 200 µM deoxyribonucleoside triphosphate, 0.4 U Taq polymerase (Gibco, Gaithersburg, MD), 0.5 µl of 5% dimethyl sulfoxide and 6.7µl H₂O. For a negative control, no DNA was added to the PCR reaction contents. The reactions were denaturated for 45 seconds at 95 °C, then DNA was amplified for 13 cycles (15 seconds at 95 °C, 30 seconds at 68 °C reduced by 1 °C per cycle and 30 seconds at 72 °C) followed by 23 cycles (15 seconds at 95 °C, 30 seconds at 55°C, 30 seconds at 72 °C) then followed by a final 5-minute extension at 72 °C. The PCR product is a 318 bp DNA fragment, 1 µl of each PCR product was electrophoresed on a 2.5 % agarose gel, stained with ethidium bromide, and visualized under UV illumination. After each PCR reaction, the remaining 9 µl of PCR product was used for enzyme digestion with 10 units *Sac II* (MBI Fermentas, Vilnius, Lithuania) in 10 mM MgCl₂, 10 mM Tris-HCl, 0.2 mg bovine serum albumin (BSA) at pH 7.5 in 9 µl purified water at 37°C overnight. Enzyme digestion revealed fragment sizes of 41, 51 and 226 bp from the A-allele and 127 and 191 bp from the B-allele, respectively. Haplotypes were confirmed by direct sequencing of PCR products from individuals with the genotypes AA, AB and BB. The digestion products were electrophoresed and visualized in the same manner as described for PCR products. They can easily be distinguished on electrophoresis.

Statistical analyses were done with SPSS (SPSS Inc., Chicago, IL). The non-parametric Pearson chi-square test was used to compare the *CST3*-BB bearers versus nonbearers consisting of the genotypes AA and AB (df=1). Kaplan-Meier survival analysis was performed on data obtained from 167 patients and 268 controls. P-values less than 0.05 were considered as statistically significant. Means and standard deviations were calculated for age of patients and controls.

### Results

The analysis of demographic data revealed that age at presentation was similar for the male and female subgroup of AMD patients and control subjects (Table 1). Both genders were separately age-matched since the number of male and female subjects among patients and controls was unequally distributed (p<0.0001). The genotype frequencies of *CST3* haplotypes were in Hardy-Weinberg equilibrium in both patients and controls.

Simultaneous genotyping of three polymorphic *SacII* restriction sites in the 5'-region of *CST3* covered by a single PCR fragment revealed a strong linkage disequilibrium between all three polymorphic *SacII* sites. The observed haplotypes were defined by either concomitant *SacII* restriction, one at 80 bp upstream of the transcription start sites and one in the penultimate codon of the sequence that encodes the signal peptide (allele A), or by exclusive cleavage downstream of the transcription start sites (allele B).

The frequency of homozygous haplotype *CST3* BB genotype differed significantly between patients with exudative AMD and control subjects (p=0.0228; Table 2). The frequency of this genotype was 0.066 for the entire patients group compared with 0.022 for control subjects overall. When subgroups for both genders were formed the frequency of the BB genotype was 0.113 in male patients and 0.024 in male controls. There was a statistically significant difference of the BB genotype distribution in the male subgroup (p=0.0119; Fisher's exact test: p=0.0201; Table 3). The frequency of the BB genotype in the female subgroup was 0.044 in patients versus 0.021 in controls and failed to differ statistically (p=0.303;) among groups (Table 4).

The effect of the BB genotype on the occurrence of exudative AMD was estimated using a odds ratio estimate (OR) in case control studies. This OR was 3.079 (95% confidence interval [CI] 1.116 to 8.490) for the entire groups of patients and controls. When the male subgroups of cases and controls were tested separately, the effect was stronger with a OR of 5.276 (95% CI 1.267 to 21.961). For the female subgroup the OR was calculated to be 2.11 (95% CI 0.493 to 9.024). Data are shown in Table 5.

Kaplan-Meier survival analysis was performed on the data obtained from patients and control subjects. The average disease-free survival time was 83 years (95 % CI = 82 - 85; S.E.=1) in the pooled *CST3* AA or AB subjects and 75 years (95 % C I= 71 - 79; S.E.=2) in *CST3* BB subjects reaching statistical significance (Mantel-Cox Log rank = 9.00; df = 1; p = 0.0027).

**Table 1:**

| **Patients with AMD and control subjects** | | |
|---|---|---|
| | Patients | Controls |
| **Sex (No.[%])** | | |
| Female | 114 (42.5) | 141 (57.5) |
| Male | 53 (31.7) | 127 (68.3) |
| **mean age at presentation** | | |
| **(yrs.**±**SD)** | | |
| Female | 75.25 ± 7.63 | 75.30 ± 7.89 |
| Male | 73.57 ± 7.41 | 73.66 ± 6.63 |

**Table 2:**

| ***CST3* genotype distribution** | | | | |
|---|---|---|---|---|
| *CST3* genotype | Patients (n=167) | | Controls (n=268) | |
| | n | % | n | % |
| AA or AB | 156 | 93.4 | 262 | 97.8 |
| BB | 11 | 6.6 | 6 | 2.2 |
| χ² = 5.18; p = 0.0228; df = 1 | | | | |

**Table 3:**

| ***CST3* genotype distribution among males** | | | | |
|---|---|---|---|---|
| *CST3* genotype | Patients (n=53) | | Controls (n=127) | |
| | n | % | n | % |
| AA or AB | 47 | 88.7 | 124 | 97.6 |
| BB | 6 | 11.3 | 3 | 2.4 |
| χ² = 6.318; p = 0.0119; df = 1; Fisher's exact test: p = 0.0202 | | | | |

**Table 4:**

| ***CST3* genotype distribution among females** | | | | |
|---|---|---|---|---|
| *CST3* genotype | Patients (n=114) | | Controls (n=141) | |
| | n | % | n | % |
| AA or AB | 109 | 95.6 | 138 | 97.9 |
| BB | 5 | 4.4 | 3 | 2.1 |
| χ² = 1.056; p = 0.303; df = 1 | | | | |

**Table 5:**

| **Odds ratios for exudative AMD with *CST3* genotype BB dependent on gender** | | |
|---|---|---|
| subgroup | Odds ratio | 95% CI |
| all patients | 3.079 | 1.117-8.49 |
| female | 2.11 | 0.493-9.024 |
| male | 5.276 | 1.267-21.961 |

### EXAMPLE 2

### Patients and methods

Patients (n = 167, age range 51 - 94 years, 114 females and 53 males, with mean ages at presentation (SD) of 75.3 (7.6) and 73.6 (7.4) years) of Example 1 were studied. The 167 patients with advanced exudative AMD represented a subset of 200 AMD patients that included patients with geographic atrophy (n = 20) or early AMD (n = 7) in one or both eyes and did not have CNV. They were therefore excluded from this study. Thus, 88 % of all AMD patients had advanced exudative AMD in at least one eye, a typical rate for our tertiary care hospital. This reflects the fact that patients with choroidal neovascularization are much more likely to suffer severe vision loss and are therefore cared for by a specialised retinal centre.

Also 517 unrelated Caucasian control subjects (age range 19 - 99 years), 283 females and 234 males with mean age of 69.5 (12.7) and 66.3 (11.5) years, respectively. In order to allow the assessment of possible regional or ethnical differences in allele frequencies, the controls consisted of an international collection of adult volunteers originating from Germany (n = 235), Switzerland (n = 164), Italy (n = 56), and USA (n = 62). The controls were not examined for ophthalomologic disorders and were expected to develop AMD at the population rate, and there were no exclusion criteria with respect to macular appearance in the control group.

Genomic D N A was isolated from peripheral blood leukocytes using a standard salt precipitation technique. PCR products (318 bp) from genomic D N A were generated by using primer 024, TGGGAGGGACGAGGCGTTCC (Balbin et al., Hum. Genet. 87(6): 751 - 752, 1991) and 1206R, TCCATGGGGCCTCCCACCAG. A 10 µl polymerase chain reaction was performed, containing 0.4 µl of suspended genomic D N A, 500 nM of each forward and reverse primer, 1.5 mM magnesium chloride, 1 µl 10 x buffer, 200 µM deoxyribonucieoside triphosphate, 0.4 units *Taq* D N A polymerase (Gibco, Gaithersburg, MD), 0.5 µl of 5 % dimethyl sulfoxide and 6.7 µl H₂O. The thermoprofile was 95 °C 45 sec., 13 x [95 °C 15 sec., 68 °C 30 sec -1 °C per cycle, 72 °C 30 sec], 23 x [95 °C 15 sec., 55 °C 30 sec, 72 °C 30 sec], and 72°C 5 min. Three polymorphic *KspI* restriction sites in the 5'-region of *CST3* were covered by the 318 pb PCR fragment. Through a strong linkage disequilibrium between the three polymorphisms only two haplotypes were observed. The haplotypes are defined by either concomitant *KspI* restriction endonuclease cleavage both 80 bp upstream of the mRNA transcription start site and in the penultimate codon of the signal peptide (haplotype A), or by an exclusive cleavage downstream of the transcription start site (haplotype B). Haplotypes were confirmed by direct sequencing of the PCR products from individuals with the genotypes A/A, A/B, and B/B. Restriction digestion of the PCR product with *KspI* (MBI Fermentas, Vilnius, Lithuania) at 37 °C overnight revealed fragment sizes of 41 / 226 / 51 bp (homozygote haplotype A), or 127 / 191 bp (homozygote haplotype B), or all five fragments in A/B heterozygotes. The digestion products were electrophoresed on a 2.5 % agarose gel, stained with ethidium bromide, and visualised under UV light.

All statistical association analyses were done with SPSS, version 8.0 (SPSS Inc., Chicago, IL). P values less than 0.05 were considered significant. Statistical analyses of deviations from Hardy Weinberg equilibrium (HWE) were done by
χ² = (X / X_{obs.} - X / X_{exp.})², where X / X_{obs.} is the observed genotype count of the respective *CST3* genotypes (A/A, A/B, or B/B) and X / X_{exp.} is the respective genotype count expected under HWE which is calculated based on the frequencies (F_{A} and F_{B}) of the observed allelic variants (here: A and B) at a given locus: F_{A} + F_{B} = 1, therefore (F_{A} + F_{B})² = 1 = (F_{A})² + (F_{B})² + 2 F_{A}F_{B}. Note: (F_{A})² = A / A_{exp.} , (F_{B})² = B / B_{exp.} , 2 F_{A}F_{B} = A / B_{exp.}.

There was no significant difference in allele frequency of haplotype B (F_{B}) in the control groups of the four centers in Germany, Switzerland, Italy and USA with F_{B} of 0.18, 0.20, 0.21, and 0.21, respectively (p = 0.22, DF = 3). The similar F_{B} between the German controls (F_{B} = 0.181) and those pooled from the other three centers with a mean F_{B} = 0.184 (p = 0.88, DF = 1) suggested widespread population similarity of F_{B} and allowed to pool all controls.

Table 6 shows the genotype counts of all patients and controls. None of the genotype counts significantly deviated from those expected under Hardy Weinberg equilibrium (HWE). There was a significant difference in genotype counts between patients and controls (χ² = 7.16, exact p = 0.028, DF = 2; two sided Fisher exact test: p = 0.037). The strongest difference between patients and controls was observed in the B/B homozygotes with 6.6 % and 2.3 %, respectively, suggesting an odds ratio (OR) for AMD in association with *CST3* B/B of 2.97 (95 % CI 1.28; 6.86). The respective proportion of A/B heterozygotes was almost identical in patients and controls. The difference in F_{B} between patients and controls with F_{B} = 0.23 and 0.18, respectively, failed to reach statistical significance (χ² = 2.83, p = 0.093, DF = 1) and may be explained by the higher proportion of B/B homozygotes in the patients. In accordance with this, there were slightly less (B/B_{obs.} = 11) than expected under HWE (B/B_{exp.} = 17.3) B/B homozygotes in the controls (χ² = 1.62) and more than expected (B/B_{obs.} = 12 vs. B/B_{exp.} = 8.4, χ² = 0.81) B/B homozygotes in the patients (Σχ² = 2.43, n.s.).

Logistic regression analysis entering *CST3* genotype (B/B vs. A/A or A/B), gender, and age revealed the highest coefficient (B) for *CST3* (B = 1.26, p = 0.005), and lower but significant B's for gnedr (B = 0.41, p = 0.038) and age (B = 0.06, p < 0.0001). Therefore the association between B/B homozygosity and AMD were reanalyzed both in separately age-matched males (53 patients, 138 controls) and females (114 patients, 211 controls). The results shown in Table 7 suggest a stronger association of B/B with AMD in males than in females.

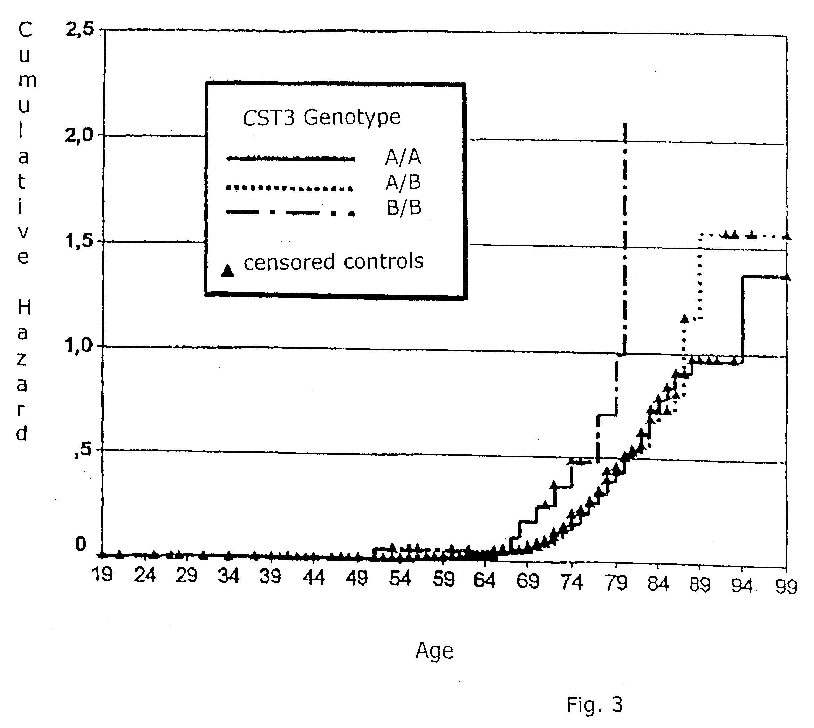

In both males and females there was a significant effect of *CST3* B/B on disease-free survival analyzed by Kaplan-Meier analysis. In males with genotypes A/A or A/B the mean disease free survival time was 86 yrs (SE 2; 95 % CI 82; 89) which was 74 yrs (SE 4; 95 % CI 67; 81) in B/B homozygotes (Log Rank p = 0.041). The mean disease free survival time in pooled males and females with genotypes A/A or A/B was 85 yrs (SE 1; 95 % CI 83; 86) and 76 yrs (SE 2; 95 % CI: 72; 79) in B/B homozygotes (Log Rank p = 0.0006). The graphic plot of the hazard function of the pooled survival analysis is shown in Figure 3. The Log Rank statistics were very similar (p= 0.0005) if a means disease onset of 3 yrs prior to clinical presentation was assumed. The oldest control subject homozygous B/B was 75 yrs, and 137 of 517 controls (26.5 %) were older than 75 yrs. The oldest patient homozygous B/B was 85, whereas only 10 of 167 patients (6 %) were older than 85.

It was decided not to choose an AMD-free population, because the distinction between early AMD and normal aging is not well defined. This study design may indeed suffer from reduced statistical power, because the control group may well include subjects with AMD as well as subjects who could manifest the disease later in life as can be expected from the normal age-dependent prevalence of AMD.

Our genotypic association data, the absence of a significant difference in allele frequencies between patients and controls, and the survival analyses show an increased susceptibility for exudative AMD in *CST3* B/B homozygotes. Therefore the *CST3* haplotype B may be a recessive risk allele, significantly contributing to disease risk in up to 6.6 % of German AMD patients.

**Table 6:**

| ***CST3* genotype counts and frequencies in patients with AMD and control subjects** | | | | |
|---|---|---|---|---|
| ***CST3* genotype counts and frequencies (%)** | | | | |
| | **N** | **A/A** | **A/B** | **B/B** |
| **Patients** | 167 | 103 (61.7) | 53 (31.7) | 11 (6.6) |
| **- Female** | 114 | 69 (60.5) | 40 (35.1) | 5 (4.4) |
| **- Male** | 53 | 34 (64.2) | 13(24.5) | 6 (11.3) |
| **Controls** | 517 | 340 (65.8) | 165 (31.9) | 12 (2.3) |
| **- Age matched, females** | 211 | 150 (71.1) | 56 (26.5) | 5 (2.4) |
| **- Age Matched, males** | 138 | 89 (64.5) | 46 (33.3) | 3 (2.2) |

**Table 7:**

| **Influence of gender and age matching of controls on odds ratio (OR) for exudative AMD (167 patients; 114 female, 53 male) in association with *CST3* genotype B/B** | | | |
|---|---|---|---|
| | **OR** | **95 % CI** | **p*** |
| **All 167 patients and 517 controls** | 2.967 | 1.284 - 6.857 | 0.013 |
| | | | |
| **females, controls age matched** | 1.890 | 0.535 - 6.670 | 0.329 |
| | | | |
| **males, controls age matched** | 5.745 | 1.328 - 23.888 | 0.015 |

| | | | |
|---|---|---|---|
| * Two sided Fisher exact test | | | |

## Claims

1. A method for diagnosing or prognosticating age-related macular degeneration in a subject, or determining whether a subject is at increased risk of developing age-related macular degeneration, comprising:
determining a level, or an activity, or both said level and said activity, of at least one substance which is selected from the group consisting of a transcription product of a Cystatin C gene, a translation product of a Cystatin C gene, a fragment of said translation product, an amyloid protein, and a transcription product of a gene coding for an amyloid protein in a sample from said subject;
and
comparing said level, or said activity, or both said level and said activity, of at least one of said substances to a reference value representing a known disease or health status,
thereby diagnosing or prognosticating said age-related macular degeneration in said subject, or determining whether said subject is at increased risk of developing age-related macular degeneration.

2. A method of monitoring the progression of age-related macular degeneration in a subject, comprising:
determining a level, or an activity, or both said level and said activity, of at least one substance which is selected from the group consisting of a transcription product of a Cystatin C gene, a translation product of a Cystatin C gene, a fragment of said translation product, an amyloid protein, and a transcription product of a gene coding for an amyloid protein in a sample from said subject;
and
comparing said level, or said activity, or both said level and said activity, of at least one of said substances to a reference value representing a known disease or health status,
thereby monitoring the progression of said age-related macular degeneration in said subject.

3. A method of evaluating a treatment for age-related macular degeneration, comprising:
determining a level, or an activity, or both said level and said activity, of at least one substance which is selected from the group consisting of a transcription product of a Cystatin C gene, a translation product of a Cystatin C gene, a fragment of said translation product, an amyloid protein, and a transcription product of a gene coding for an amyloid protein, in a sample obtained from a subject being treated for said age-related macular degeneration;
and
comparing said level, or said activity, or both said level and said activity, of at least one of said substances to a reference value representing a known disease or health status,
thereby evaluating said treatment for said age-related macular degeneration.

4. The method according to at least one of claims 1 to 3, wherein said sample is taken from a body fluid, a tissue, or an organ, in particular an eye, of said subject.

5. The method according to claim 4, wherein said sample is taken from material located between the plasma membrane and basal lamina of the retinal pigment epithelium.

6. The method according to claim 4, wherein said sample is taken from material located between the basal lamina of the retinal pigment epithelium and the inner collagenous zone of Bruch's membrane.

7. The method according to at least one of claims 1 to 6, wherein a variation of said level of Cystatin C or a transcription product of a Cystatin C gene in said sample from said subject relative to said reference value representing a known health status indicates a diagnosis, or prognosis, or increased risk of said age-related macular degeneration in said subject.

8. The method according to at least one of claims 1 to 7, wherein a varied activity of Cystatin C in said sample from said subject relative to said reference value representing a known health status indicates a diagnosis, or prognosis, or increased risk of said age-related macular degeneration in said subject.

9. The method according to at least one of claims 1 to 8, wherein said Cystatin C gene is a polymorphic variant of the Cystatin C wild-type gene.

10. The method according to claim 9, wherein the presence of at least one B allele indicates said subject is at increased risk of developing age-related macular degeneration or indicates a diagnosis or prognosis of age-related macular degeneration.

11. The method according to claim 10, wherein the presence of the B/B genotype indicates said subject is at increased risk of developing age-related macular degeneration or indicates a diagnosis or prognosis of age-related macular degeneration.

12. The method according to at least one of claims 1 to 11, wherein said subject is a human.

13. The method according to at least one of claims 1 to 12, wherein said Cystatin C is determined in its monomeric form.

14. The method according to at least one of claims 1 to 13, wherein at least one of said substances is detected using an immunoassay, an enzyme activity assay and/or a binding assay.

15. The method according to at least one of claims 1 to 14, wherein said reference value is that of a level, or an activity, or both said level and said activity, of at least one substance which is selected from the group consisting of a transcription product of a Cystatin C gene, a translation product of a Cystatin C gene, a fragment of said translation product, an amyloid protein, and a transcription product of a gene coding for an amyloid protein in a sample from a subject not suffering from said age-related macular degeneration.

16. The method according to at least one of claims 1 to 15, further comprising comparing a level, or an activity, or both said level and said activity, of at least one substance which is selected from the group consisting of a transcription product of a Cystatin C gene, a translation product of a Cystatin C gene, a fragment of said translation product, an amyloid protein, a transcription product of a gene coding for an amyloid protein in said sample with a level, an activity, or both said level level and said activity, of at least one of said substances in a series of samples taken from said subject over a period of time.

17. The method according to at least one of claims 1 to 16, wherein said subject receives a treatment prior to one or more of said sample gatherings.

18. The method according to claim 17, wherein said level, or said activity, or both said level and said activity, in said samples is determined, before and after said treatment is administered to said subject.

19. A method of diagnosing or prognosticating age-related macular degeneration in a subject, or determining whether a subject is at increased risk of developing age-related macular degeneration comprising:
determining a presence or absence of a mutation or polymorphism in a Cystatin C gene in a sample from said subject,
thereby diagnosing or prognosticating age-related macular degeneration in said subject, or determining whether said subject is at increased risk of developing age-related macular degeneration.

20. The method of claim 19, wherein the presence or absence of at least one B allele is determined.

21. The method of claim 20, wherein the presence of at least one 8 allele, in particular the presence of the B/B genotype, indicates said subject is at increased risk of developing age-related macular degeneration or indicates a diagnosis or prognosis of age-related macular degeneration.

22. The method of at least one of claims 19 to 21, further comprising:
determining a level, or an activity, or both said level and said activity, of at least one substance which is selected from the group consisting of a transcription product of a Cystatin C gene, a translation product of a Cystatin C gene, a fragment of said translation product, an amyloid protein, and a transcription product of a gene coding for an amyloid protein, in a sample from said subject;
and
comparing said level, or said activity, or both said level and said activity, of at least one of said substances to a reference value representing a known disease or health status.

23. The method according to claim 22, wherein a variation of said level of Cystatin C or a transcription product of a Cystatin C gene in said sample from said subject relative to said reference value representing a known health status indicates a diagnosis, or prognosis, or increased risk of said age-related macular degeneration in said subject.

24. The method according to claim 22 or 23, wherein a varied activity of Cystatin C in said sample from said subject relative to said reference value representing a known health status indicates a diagnosis, or prognosis, or increased risk of said age-related macular degeneration in said subject.

25. Use of a kit for diagnosis or prognosis of age-related macular degeneration, or for determination of increased risk of developing age-related macular degeneration, or for monitoring progression of age-related macular degeneration in said subject, or for monitoring success or failure of a therapeutic treatment of said subject, said kit comprising at least one reagent which is selected from the group consisting of (i) reagents that selectively detect a transcription product and/or a translation product of a Cystatin C gene, (ii) reagents that selectively detect a fragment of a translation product of a Cystatin C gene, (iii) reagents that selectively detect a mutation or polymorphism in a Cystatin C gene, and (iv) reagents that selectively detect a transcription product and/or a translation product of a gene coding for an amyloid protein.

26. The use according to claim 25 wherein said reagents selectively detect a polymorphic variant of the wild-type Cystatin C gene.

27. The use according to claim 26 wherein said reagents selectively detect a B allele of the Cystatin C gene.

28. The use according to at least one of claims 25 to 27 for working the methods according to claims 1 to 24.

## Patentansprüche

1. Verfahren zum Diagnostizieren oder Prognostizieren einer altersbedingten Makuladegeneration bei einem Patienten oder zum Bestimmen, ob ein Patient ein erhöhtes Risiko hat, eine altersbedingte Makuladegeneration zu entwickeln, umfassend:
Bestimmen einer Konzentration oder einer Aktivität oder sowohl einer Konzentration als auch einer Aktivität wenigstens einer Substanz, die aus der Gruppe ausgewählt ist, die aus einem Transcriptionsprodukt eines Cystatin-C-Gens, einem Translationsprodukt eines Cystatin-C-Gens, einem Fragment des Translationsprodukts, einem Amyloidprotein und einem Transcriptionsprodukt eines Gens, das für ein Amyloidprotein codiert, besteht, in einer Probe von dem Patienten; und
Vergleichen der Konzentration oder der Aktivität oder sowohl der Konzentration als auch der Aktivität von wenigstens einer der Substanzen mit einem Referenzwert, der einen bekannten Krankheits- oder Gesundheitszustand darstellt;
wodurch die altersbedingte Makuladegeneration bei dem Patienten diagnostiziert oder prognostiziert wird oder bestimmt wird, ob der Patient ein erhöhtes Risiko hat, eine altersbedingte Makuladegeneration zu entwickeln.

2. Verfahren zur Überwachung des Fortschritts von altersbedingter Makuladegeneration bei einem Patienten, umfassend:
Bestimmen einer Konzentration oder einer Aktivität oder sowohl einer Konzentration als auch einer Aktivität wenigstens einer Substanz, die aus der Gruppe ausgewählt ist, die aus einem Transcriptionsprodukt eines Cystatin-C-Gens, einem Translationsprodukt eines Cystatin-C-Gens, einem Fragment des Translationsprodukts, einem Amyloidprotein und einem Transcriptionsprodukt eines Gens, das für ein Amyloidprotein codiert, besteht, in einer Probe von dem Patienten; und
Vergleichen der Konzentration oder der Aktivität oder sowohl der Konzentration als auch der Aktivität von wenigstens einer der Substanzen mit einem Referenzwert, der einen bekannten Krankheits- oder Gesundheitszustand darstellt;
wodurch der Fortschritt der altersbedingten Makuladegeneration bei dem Patienten überwacht wird.

3. Verfahren zur Überwachung einer Behandlung auf altersbedingte Makuladegeneration, umfassend:
Bestimmen einer Konzentration oder einer Aktivität oder sowohl einer Konzentration als auch einer Aktivität wenigstens einer Substanz, die aus der Gruppe ausgewählt ist, die aus einem Transcriptionsprodukt eines Cystatin-C-Gens, einem Translationsprodukt eines Cystatin-C-Gens, einem Fragment des Translationsprodukts, einem Amyloidprotein und einem Transcriptionsprodukt eines Gens, das für ein Amyloidprotein codiert, besteht, in einer Probe, die von einem Patienten erhalten wurde, der auf die altersbedingte Makuladegeneration behandelt wird; und
Vergleichen der Konzentration oder der Aktivität oder sowohl der Konzentration als auch der Aktivität von wenigstens einer der Substanzen mit einem Referenzwert, der einen bekannten Krankheits- oder Gesundheitszustand darstellt;
wodurch die Behandlung auf altersbedingte Makuladegeneration überwacht wird.

4. Verfahren gemäß wenigstens einem der Ansprüche 1 bis 3, wobei die Probe aus einer Körperflüssigkeit, einem Gewebe oder einem Organ, insbesondere einem Auge, des Patienten entnommen wird.

5. Verfahren gemäß Anspruch 4, wobei die Probe aus Material entnommen wird, das sich zwischen der Plasmamembran und der Basalmembran des retinalen Pigmentepithels befindet.

6. Verfahren gemäß Anspruch 4, wobei die Probe aus Material entnommen wird, das sich zwischen der Basalmembran des retinalen Pigmentepithels und der inneren Kollagenschicht der Bruchschen Membran befindet.

7. Verfahren gemäß wenigstens einem der Ansprüche 1 bis 6, wobei eine Variation der Konzentration von Cystatin C oder eines Transcriptionsprodukts eines Cystatin-C-Gens in der Probe von dem Patienten relativ zu dem Referenzwert, der einen bekannten Gesundheitszustand darstellt, eine Diagnose oder Prognose oder ein erhöhtes Risiko der altersbedingten Makuladegeneration bei dem Patienten anzeigt.

8. Verfahren gemäß wenigstens einem der Ansprüche 1 bis 7, wobei eine variierte Aktivität von Cystatin C in der Probe von dem Patienten relativ zu dem Referenzwert, der einen bekannten Gesundheitszustand darstellt, eine Diagnose oder Prognose oder ein erhöhtes Risiko der altersbedingten Makuladegeneration bei dem Patienten anzeigt.

9. Verfahren gemäß wenigstens einem der Ansprüche 1 bis 8, wobei das Cystatin-C-Gen eine polymorphe Variante des Cystatin-C-Wildtyp-Gens ist.

10. Verfahren gemäß Anspruch 9, wobei die Anwesenheit von wenigstens einem B-Allel anzeigt, dass der Patient ein erhöhtes Risiko hat, eine altersbedingte Makuladegeneration zu entwickeln, oder eine Diagnose oder Prognose der altersbedingten Makuladegeneration anzeigt.

11. Verfahren gemäß Anspruch 10, wobei die Anwesenheit des B/B-Genotyps anzeigt, dass der Patient ein erhöhtes Risiko hat, eine altersbedingte Makuladegeneration zu entwickeln, oder eine Diagnose oder Prognose der altersbedingten Makuladegeneration anzeigt.

12. Verfahren gemäß wenigstens einem der Ansprüche 1 bis 11, wobei der Patient ein Mensch ist.

13. Verfahren gemäß wenigstens einem der Ansprüche 1 bis 12, wobei das Cystatin C in seiner monomeren Form bestimmt wird.

14. Verfahren gemäß wenigstens einem der Ansprüche 1 bis 13, wobei wenigstens eine der Substanzen unter Verwendung eines Immunoassays, eines Enzymaktivitätsassays und/oder eines Bindungsassays nachgewiesen wird.

15. Verfahren gemäß wenigstens einem der Ansprüche 1 bis 14, wobei der Referenzwert der Referenzwert einer Konzentration oder einer Aktivität oder sowohl einer Konzentration als auch einer Aktivität wenigstens einer Substanz, die aus der Gruppe ausgewählt ist, die aus einem Transcriptionsprodukt eines Cystatin-C-Gens, einem Translationsprodukt eines Cystatin-C-Gens, einem Fragment des Translationsprodukts, einem Amyloidprotein und einem Transcriptionsprodukt eines Gens, das für ein Amyloidprotein codiert, besteht, in einer Probe von einem Patienten, der nicht an der altersbedingten Makuladegeneration leidet, ist.

16. Verfahren gemäß wenigstens einem der Ansprüche 1 bis 15, das weiterhin folgendes umfasst: Vergleichen einer Konzentration oder einer Aktivität oder sowohl einer Konzentration als auch einer Aktivität von wenigstens einer Substanz, die aus der Gruppe ausgewählt ist, die aus einem Transcriptionsprodukt eines Cystatin-C-Gens, einem Translationsprodukt eines Cystatin-C-Gens, einem Fragment des Translationsprodukts, einem Amyloidprotein und einem Transcriptionsprodukt eines Gens, das für ein Amyloidprotein codiert, besteht, in der Probe mit einer Konzentration oder einer Aktivität oder sowohl einer Konzentration als auch einer Aktivität von wenigstens einer der Substanzen in einer Reihe von Proben, die über einen Zeitraum hinweg von dem Patienten entnommen wurden.

17. Verfahren gemäß wenigstens einem der Ansprüche 1 bis 16, wobei der Patient vor einer oder mehreren der Probeentnahmen eine Behandlung erhält.

18. Verfahren gemäß Anspruch 17, wobei die Konzentration oder die Aktivität oder sowohl die Konzentration als auch die Aktivität in den Proben bestimmt wird, bevor und nachdem die Behandlung dem Patienten verabreicht wird.

19. Verfahren zum Diagnostizieren oder Prognostizieren einer altersbedingten Makuladegeneration bei einem Patienten oder zum Bestimmen, ob ein Patient ein erhöhtes Risiko hat, eine altersbedingte Makuladegeneration zu entwickeln, umfassend:
Bestimmen einer Anwesenheit oder Abwesenheit einer Mutation oder eines Polymorphismus in einem Cystatin-C-Gen in einer Probe von dem Patienten;
wodurch die altersbedingte Makuladegeneration bei dem Patienten diagnostiziert oder prognostiziert wird oder bestimmt wird, ob der Patient ein erhöhtes Risiko hat, eine altersbedingte Makuladegeneration zu entwickeln.

20. Verfahren gemäß Anspruch 19, wobei die Anwesenheit oder Abwesenheit wenigstens eines B-Allels bestimmt wird.

21. Verfahren gemäß Anspruch 20, wobei die Anwesenheit wenigstens eines B-Allels, insbesondere die Anwesenheit des B/B-Genotyps, anzeigt, dass der Patient ein erhöhtes Risiko hat, eine altersbedingte Makuladegeneration zu entwickeln, oder eine Diagnose oder Prognose der altersbedingten Makuladegeneration anzeigt.

22. Verfahren gemäß wenigstens einem der Ansprüche 19 bis 21, das weiterhin folgendes umfasst: Vergleichen einer Konzentration oder einer Aktivität oder sowohl einer Konzentration als auch einer Aktivität von wenigstens einer Substanz, die aus der Gruppe ausgewählt ist, die aus einem Transcriptionsprodukt eines Cystatin-C-Gens, einem Translationsprodukt eines Cystatin-C-Gens, einem Fragment des Translationsprodukts, einem Amyloidprotein und einem Transcriptionsprodukt eines Gens, das für ein Amyloidprotein codiert, besteht, in einer Probe von dem Patienten; und
Vergleichen der Konzentration oder der Aktivität oder sowohl der Konzentration als auch der Aktivität von wenigstens einer der Substanzen mit einem Referenzwert, der einen bekannten Krankheits- oder Gesundheitszustand darstellt.

23. Verfahren gemäß Anspruch 22, wobei eine Variation der Konzentration von Cystatin C oder eines Transcriptionsprodukts eines Cystatin-C-Gens in der Probe von dem Patienten relativ zu dem Referenzwert, der einen bekannten Gesundheitszustand darstellt, eine Diagnose oder Prognose oder ein erhöhtes Risiko der altersbedingten Makuladegeneration bei dem Patienten anzeigt.

24. Verfahren gemäß Anspruch 22 oder 23, wobei eine variierte Aktivität von Cystatin C in der Probe von dem Patienten relativ zu dem Referenzwert, der einen bekannten Gesundheitszustand darstellt, eine Diagnose oder Prognose oder ein erhöhtes Risiko der altersbedingten Makuladegeneration bei dem Patienten anzeigt.

25. Verwendung eines Kits für die Diagnose oder Prognose von altersbedingter Makuladegeneration oder zur Bestimmung eines erhöhten Risikos, eine altersbedingte Makuladegeneration zu entwickeln, oder für die Überwachung des Fortschritts von altersbedingter Makuladegeneration bei einem Patienten oder für die Überwachung des Erfolgs oder Misserfolgs einer therapeutischen Behandlung des Patienten, wobei der Kit folgendes umfasst: wenigstens ein Reagens, das aus der Gruppe ausgewählt ist, die aus Folgendem besteht: (i) Reagentien, die selektiv ein Transcriptionsprodukt und/oder ein Translationsprodukt eines Cystatin-C-Gens nachweisen, (ii) Reagentien, die selektiv ein Fragment eines Translationsprodukts eines Cystatin-C-Gens nachweisen, (iii) Reagentien, die selektiv eine Mutation oder einen Polymorphismus in einem Cystatin-C-Gen nachweisen, und (iv) Reagentien, die selektiv ein Transcriptionsprodukt und/oder ein Translationsprodukt eines Gens, das für ein Amyloidprotein codiert, nachweisen.

26. Verwendung gemäß Anspruch 25, wobei die Reagentien selektiv eine polymorphe Variante des Wildtyp-Cystatin-C-Gens nachweisen.

27. Verwendung gemäß Anspruch 26, wobei die Reagentien selektiv ein B-Allel des Cystatin-C-Gens nachweisen.

28. Verwendung gemäß wenigstens einem der Ansprüche 25 bis 27 zur Durchführung der Verfahren gemäß den Ansprüchen 1 bis 24.

## Revendications

1. Procédé pour diagnostiquer ou pronostiquer une dégénérescence maculaire liée à l'âge chez un sujet, ou déterminer si un sujet présente un risque accru de développer une dégénérescence maculaire liée à l'âge, comprenant :
la détermination d'un taux, ou d'une activité, ou à la fois dudit taux et de ladite activité, d'au moins une substance qui est choisie dans le groupe constitué par un produit de transcription d'un gène de cystatine C, un produit de traduction d'un gène de cystatine C, un fragment dudit produit de traduction, une protéine amyloïde et un produit de transcription d'un gène codant pour une protéine amyloïde dans un échantillon dudit sujet ; et
la comparaison dudit taux, ou de ladite activité, ou à la fois dudit taux et de ladite activité, d'au moins l'une desdites substances avec une valeur de référence représentant une maladie ou un état de santé connu(e),
de façon à diagnostiquer ou pronostiquer ladite dégénérescence maculaire liée à l'âge chez ledit sujet, ou à déterminer si ledit sujet présente un risque accru de développer une dégénérescence maculaire liée à l'âge.

2. Procédé pour suivre la progression d'une dégénérescence maculaire liée à l'âge chez un sujet, comprenant :
la détermination d'un taux, ou d'une activité, ou à la fois dudit taux et de ladite activité, d'au moins une substance qui est choisie dans le groupe constitué par un produit de transcription d'un gène de cystatine C, un produit de traduction d'un gène de cystatine C, un fragment dudit produit de traduction, une protéine amyloïde et un produit de transcription d'un gène codant pour une protéine amyloïde dans un échantillon dudit sujet ; et
la comparaison dudit taux, ou de ladite activité, ou à la fois dudit taux et de ladite activité, d'au moins l'une desdites substances avec une valeur de référence représentant une maladie ou un état de santé connu(e),
de façon à suivre la progression de ladite dégénérescence maculaire liée à l'âge chez ledit sujet.

3. Procédé d'évaluation d'un traitement pour une dégénérescence maculaire liée à l'âge, comprenant :
la détermination d'un taux, ou d'une activité, ou à la fois dudit taux et de ladite activité, d'au moins une substance qui est choisie dans le groupe constitué par un produit de transcription d'un gène de cystatine C, un produit de traduction d'un gène de cystatine C, un fragment dudit produit de traduction, une protéine amyloïde et un produit de transcription d'un gène codant pour une protéine amyloïde, dans un échantillon obtenu auprès d'un sujet qui est traité pour ladite dégénérescence maculaire liée à l'âge ; et
la comparaison dudit taux, ou de ladite activité, ou à la fois dudit taux et de ladite activité, d'au moins l'une desdites substances avec une valeur de référence représentant une maladie ou un état de santé connu(e),
de façon à évaluer ledit traitement de ladite dégénérescence maculaire liée à l'âge.

4. Procédé selon au moins l'une des revendications 1 à 3, dans lequel ledit échantillon est prélevé d'un fluide corporel, d'un tissu ou d'un organe, en particulier un oeil, dudit sujet.

5. Procédé selon la revendication 4, dans lequel ledit échantillon est prélevé d'une matière située entre la membrane plasmatique et la lame basale de l'épithélium pigmentaire rétinien.

6. Procédé selon la revendication 4, dans lequel ledit échantillon est prélevé d'une matière située entre la lame basale de l'épithélium pigmentaire rétinien et la zone collagène interne de la membrane de Bruch.

7. Procédé selon au moins l'une des revendications 1 à 6, dans lequel une variation dudit taux de cystatine C ou d'un produit de transcription d'un gène de cystatine C dans ledit échantillon dudit sujet par rapport à ladite valeur de référence représentant un état de santé connu indique un diagnostic, ou un pronostic, ou un risque accru de dégénérescence maculaire liée à l'âge chez ledit sujet.

8. Procédé selon au moins l'une des revendications 1 à 7, dans lequel une variation d'activité de la cystatine C dans ledit échantillon dudit sujet par rapport à ladite valeur de référence représentant un état de santé connu indique une diagnostic, ou un pronostic, ou un risque accru de ladite dégénérescence maculaire liée à l'âge chez ledit sujet.

9. Procédé selon au moins l'une des revendications 1 à 8, dans lequel ledit gène de cystatine C est une variante polymorphe du gène de cystatine C de type sauvage.

10. Procédé selon la revendication 9, dans lequel la présence d'au moins un allèle B indique que ledit sujet présente un risque accru de développer une dégénérescence maculaire liée à l'âge ou indique un diagnostic ou pronostic d'une dégénérescence maculaire liée à l'âge.

11. Procédé selon la revendication 10, dans lequel la présence du génotype BIB indique que ledit sujet présente un risque accru de développer une dégénérescence maculaire liée à l'âge ou indique un diagnostic ou un pronostic d'une dégénérescence maculaire liée à l'âge.

12. Procédé selon au moins l'une des revendications 1 à 11, dans lequel ledit sujet est un humain.

13. Procédé selon au moins l'une des revendications 1 à 12, dans lequel ladite cystatine C est déterminée sous sa forme monomère.

14. Procédé selon au moins l'une des revendications 1 à 13, dans lequel au moins l'une desdites substances est détectée en utilisant un immunoessai, un essai d'activité enzymatique et/ou un essai de liaison.

15. Procédé selon au moins l'une des revendications 1 à 14, dans lequel ladite valeur de référence est celle d'un taux, ou d'une activité, ou à la fois dudit taux et de ladite activité, d'au moins une substance qui est choisie dans le groupe constitué par un produit de transcription d'un gène de cystatine C, un produit de traduction d'un gène de cystatine C, un fragment dudit produit de traduction, une protéine amyloïde et un produit de transcription d'un gène codant pour une protéine amyloïde dans un échantillon d'un sujet ne souffrant pas de ladite dégénérescence maculaire liée à l'âge.

16. Procédé selon au moins l'une des revendications 1 à 15, comprenant en outre la comparaison d'un taux, ou d'une activité, ou à la fois dudit taux et de ladite activité, d'au moins une substance qui est choisie dans le groupe constitué par un produit de transcription d'un gène de cystatine C, un produit de traduction d'un gène de cystatine C, un fragment dudit produit de traduction, une protéine amyloïde et un produit de transcription d'un gène codant pour une protéine amyloïde dans ledit échantillon, avec un taux, une activité, ou à la fois ledit taux et ladite activité, d'au moins l'une desdites substances dans une série d'échantillons prélevés dudit sujet sur une période de temps.

17. Procédé selon au moins l'une des revendications 1 à 16, dans lequel ledit sujet reçoit un traitement avant un ou plusieurs desdits prélèvements d'échantillon.

18. Procédé selon la revendication 17, dans lequel ledit taux, ou ladite activité, ou à la fois ledit taux et ladite activité, dans lesdits échantillons est déterminé, avant et après l'administration dudit traitement audit sujet.

19. Procédé pour diagnostiquer ou pronostiquer une dégénérescence maculaire liée à l'âge chez un sujet, ou déterminer si un sujet présente un risque accru de développer une dégénérescence maculaire liée à l'âge, comprenant :
la détermination de la présence ou de l'absence d'une mutation ou d'un polymorphisme dans un gène de cystatine C dans un échantillon dudit sujet,
de façon à diagnostiquer ou pronostiquer ladite dégénérescence maculaire liée à l'âge chez ledit sujet, ou à déterminer si ledit sujet présente un risque accru de développer une dégénérescence maculaire liée à l'âge.

20. Procédé selon la revendication 19, dans lequel la présence ou l'absence d'au moins un allèle B est déterminée.

21. Procédé selon la revendication 20, dans lequel la présence d'au moins un allèle B, en particulier la présence du génotype B/B, indique que ledit sujet présente un risque accru de développer une dégénérescence maculaire liée à l'âge ou indique un diagnostic ou un pronostic d'une dégénérescence maculaire liée à l'âge.

22. Procédé selon au moins l'une des revendications 19 à 21, comprenant en outre :
la détermination d'un taux, ou d'une activité, ou à la fois dudit taux et de ladite activité, d'au moins une substance qui est choisie dans le groupe constitué par un produit de transcription d'un gène de cystatine C, un produit de traduction d'un gène de cystatine C, un fragment dudit produit de traduction, une protéine amyloïde et un produit de transcription d'un gène codant pour une protéine amyloïde, dans un échantillon dudit sujet ; et
la comparaison dudit taux, ou de ladite activité, ou à la fois dudit taux et de ladite activité, d'au moins l'une desdites substances avec une valeur de référence représentant une maladie ou un état de santé connu(e).

23. Procédé selon la revendication 22, dans lequel une variation dudit taux de cystatine C ou d'un produit de transcription d'un gène de cystatine C dans ledit échantillon dudit sujet par rapport à ladite valeur de référence représentant un état de santé connu indique un diagnostic, ou un pronostic, ou un risque accru de ladite dégénérescence maculaire liée à l'âge chez ledit sujet.

24. Procédé selon la revendication 22 ou 23, dans lequel une variation d'activité de la cystatine C dans ledit échantillon dudit sujet par rapport à ladite valeur de référence représentant un état de santé connu indique un diagnostic, ou un pronostic, ou un risque accru de ladite dégénérescence maculaire liée à l'âge chez ledit sujet.

25. Utilisation d'un nécessaire pour le diagnostic ou le pronostic d'une dégénérescence maculaire liée à l'âge, ou pour la détermination d'un risque accru de développer une dégénérescence maculaire liée à l'âge, ou pour le suivi de la progression d'une dégénérescence maculaire liée à l'âge chez ledit sujet, ou pour l'observation de la réussite ou de l'échec d'un traitement thérapeutique dudit sujet, ledit nécessaire comprenant au moins un réactif qui est choisi dans le groupe constitué par (i) les réactifs qui détectent sélectivement un produit de transcription et/ou un produit de traduction d'un gène de cystatine C, (ii) les réactifs qui détectent sélectivement un fragment d'un produit de traduction d'un gène de cystatine C, (iii) les réactifs qui détectent sélectivement une mutation ou un polymorphisme dans un gène de cystatine C, et (iv) les réactifs qui détectent sélectivement un produit de transcription et/ou un produit de traduction d'un gène codant pour une protéine amyloïde.

26. Utilisation selon la revendication 25, dans laquelle lesdits réactifs détectent sélectivement une variante polymorphe du gène de cystatine C de type sauvage.

27. Utilisation selon la revendication 26, dans laquelle lesdits réactifs détectent sélectivement un allèle B du gène de cystatine C.

28. Utilisation selon au moins l'une des revendications 25 à 27 pour mettre en oeuvre les procédés selon les revendications 1 à 24.
